# EUROPÄISCHE PATENTSCHRIFT

(11) **EP 1 432 428 B1**
(45) Veröffentlichungstag und Bekanntmachung des Hinweises auf die Patenterteilung: **02.11.2006**
(21) Anmeldenummer: 02774646.0
(22) Anmeldetag: 25.09.2002
(51) Int. Cl.: A61K 35/00

(54) **Mittel zum Färben von keratinhaltigen Fasern**
Agent for colouring fibres containing keratin
Agent pour teindre des fibres contenant de la kératine

(30) Priorität: 04.10.2001 DE 10148841
(43) Veröffentlichungstag der Anmeldung: 30.06.2004
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE)
(86) Internationale Anmeldenummer: PCT/EP2002/010734
(87) Internationale Veröffentlichungsnummer: WO 2003/030842

(56) Entgegenhaltungen:
- EP-A- 0 604 278
- EP-A- 0 780 118
- EP-A- 0 873 745
- WO-A-99/66890

## Beschreibung

Die Erfindung betrifft ein Mittel zum Färben von keratinhaltigen Fasern, insbesondere menschlichen Haaren, das aromatische oder heteroaromatische Aldehyde und/oder Ketone in Kombination mit 1-Acylindolin-3-onen enthält, die Verwendung dieser Kombination in Mitteln zum Färben von keratinhaltigen Fasern sowie ein Verfahren zum Färben von keratinhaltigen Fasern, insbesondere menschlichen Haaren.

Für das Färben von keratinhaltigen Fasern, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2'-hydroxyethylamino)-anisol (Lehmanns Blau), 1-Phenyl-3-methyl-pyrazol-5-on, 2,4-Dichlor-3-aminophenol, 1,3-Bis-(2',4'-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Die Verwendung der unten näher beschriebenen Kombination aus aromatischen oder heteroaromatischen Aldehyden und/oder Ketonen und 1-Acylindolin-3-onen zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass eine Kombination aus aromatischen oder heteroaromatischen Aldehyden und/oder Ketonen mit der Formel I sowie den in der Formel II dargestellten 1-Acylindolin-3-onen sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden insbesondere Ausfärbungen über einen Nuancenbereich von gelb über gelbbraun, orange, braunorange, rot, rotbraun, violett bis hin zu blauviolett und schwarz erhalten. Der Einsatz von oxidierenden Agentien soll jedoch nicht prinzipiell ausgeschlossen werden.

Gegenstand der Erfindung ist ein Mittel zum Färben von keratinhaltigen Fasern, insbesondere menschlichen Haaren, enthaltend
**A**. mindestens einen Aldehyd und/oder ein Keton, ausgewählt aus Verbindungen der Formeln Ia und Ib, und/oder deren funktionelle Carbonylderivate und/oder physiologisch verträgliche Salze davon, 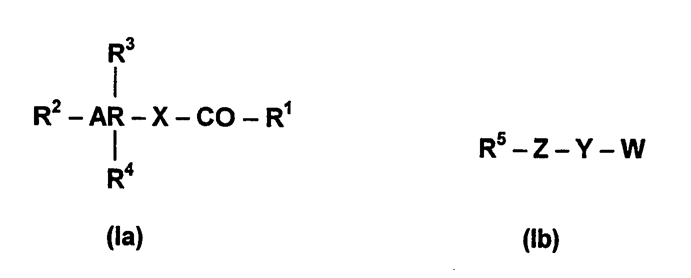 wobei
   - AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
   - R¹ steht für ein Wasserstoffatom, eine C₁-C₄-Alkyl-, C₁-C₄-Acyl-, C₂-C₄₋Alkenyl-, C₁-C₄-Pertluoralkyl-, eine ggf. substituierte Aryl- oder Heteroarylgruppe,
   - R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄₋Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₁-C₄-Hydroxyalkoxygruppen, C₂₋₄₋Alkenylgruppen, mit ggf. substituierten Benzylgruppen, mit Sulfo-(C₁₋₄)-alkyl- oder Heterozyklus-(C₁₋₄)-alkylgruppen substituiert sein können, wobei auch zwei der Reste -X-CO-R¹, R², R³ und R⁴ zusammen einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
   - X steht für eine direkte Bindung, eine Carbonyl-, Methylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
   - R⁵ steht für ein Wasserstoffatom oder eine C₁-C₄-Alkylgruppe,
   - Z steht für eine Gruppe 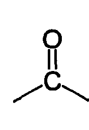 oder eine Gruppe 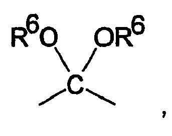 in der R⁶ eine C₁-C₄-Alkylgruppe bedeutet,
   - Y steht für eine direkte Bindung, eine C₁-C₄-Alkylengruppe, eine ggf. substituierte C₂-C₄-Alkenylen- oder C₄-C₆-Alkadienylengruppe oder eine Gruppe CHOR^{6a}; in der R^{6a} ein Wasserstoffatom oder eine C₁-C₄-Alkylgruppe bedeutet,
   - W steht für ein Wasserstoffatom, eine C₁-C₄-Alkoxygruppe, eine Formylgruppe oder eine Gruppe 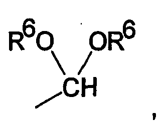 in der R⁶ eine C₁-C₄₋Alkylgruppe bedeutet,
B. mindestens ein 1-Acylindolin-3-on mit der Formel II oder ein Derivat davon oder deren physiologisch verträglichen Salze, 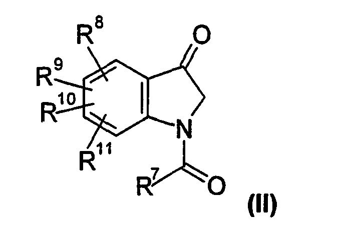 wobei
   - R⁷ steht für ein Wasserstoffatom, eine C₁-C₄-Alkyl-, eine Aryl-, eine Aryl-C₁₋C₄-alkyl- oder eine Heteroarylgruppe und
   - R⁸, R⁹, R¹⁰ und R¹¹ stehen jeweils unabhängig voneinander für ein Wasserstoffatom, ein Halogenatom, eine C₁-C₄-Alkyl-, C₁-C₄-Alkoxy- oder C₁₋C₄-Hydroxyalkoxygruppe, eine Hydroxy-, Nitro-, Carboxy-, Carboxylato-, C₁₋C₄-Alkoxycarbonyl-, eine gegebenenfalls substituierte Carbamoyl-, Sulfo-, Sulfato-, Sulfamoyl- oder Aminogruppe, die durch C₁-C₄-Alkyl- oder C₁-C₄₋Hydroxyalkylgruppen substituiert sein kann.

Die Verbindungen mit der Formel la oder Ib können auch in Form ihrer Hydrate, Acetale oder Azomethine vorliegen. Als weitere Derivate der Verbindungen der Formeln la und lb können die Carboxylate und Sulfonate, vorzugsweise mit Alkali-, Erdalkali- und Ammoniumionen als Gegenionen, genannt werden.

Geeignete Derivate der Verbindungen mit der Formel II sind Enole, Enolate sowie deren O-Acylierungs- und O-Alkylierungsprodukte gemäß Formel III, 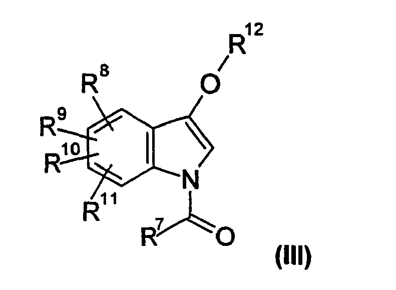 worin
- R⁷, R⁸, R⁹, R¹⁰ und R¹¹ wie in Formel II definiert sind und
- R¹² steht für ein Wasserstoffatom, eine C₁-C₄-Alkylgruppe oder eine C₁-C₄₋Acylgruppe.

Enthalten die Verbindungen mit den Formeln Ia, Ib und II N-Heteroaromaten oder Aminogruppen, können deren N-Atome quaterniert sein, z.B. durch durch C₁-C₄₋Alkyl-, Aryl-C₁-C₄-alkyl-, C₁-C₄-Suffoalkyl-, C₁-C₄-Carboxyalkyl-, C₁-C₄₋Hydroxyalkyl-, C₂-C₆-Alkenylgruppen, die ggf. substituiert sein können, oder eine Oxidogruppe. Als Gegenionen der quaternierten heteroaromatischen Carbonylverbindungen können Halogenide, wie Chlorid, Bromid oder lodid, Benzolsulfonat, p-Toluolsulfonat, C₁-C₄-Alkansulfonat, wie Methansulfonat oder Ethansulfonat, Trifluormethansulfonat, Perchlorat, Sulfat, Hydrogensulfat, Tetrafluoroborat, Phosphat, Hexafluorophosphat oder Tetrachlorozinkat genannt werden.

Beispiele für die als Substituenten im Rahmen dieser Anmeldung genannten C₁₋C₄-Alkylreste sind die Gruppen Methyl, Ethyl, Propyl, Isopropyl, n-Butyl, sec-Butyl und tert.-Butyl. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 1,2-Dihydroxyethylgruppe und die 2,3-Dihydroxypropylgruppe genannt werden. Eine 2-Hydroxyethylgruppe und eine 2,3-Dihydroxypropylgruppe sind besonders bevorzugt. Ein bevorzugter C₁₋C₄-Perfluoralkylrest ist die Trifluormethylgruppe. Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für C₁-C₄₋Alkoxycarbonylgruppen; die Methoxycarbonyl- und die Ethoxycarbonylgruppe sind dabei besonders bevorzugt. Eine bevorzugte Hydroxy-C₁-C₄-Alkoxygruppe ist die 2-Hydroxyethoxygruppe. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Beispiele für eine Heterozyklus-C₁₋₄-alkylgruppe sind Pyrrolidino-(C₁₋₄)-alkyl, Piperidino-(C₁₋₄)-alkyl, Morpholino-(C₁₋₄)-alkyl, 2-Furyl-(C₁₋₄)-alkyl, 2-Thienyl-(C₁₋₄)-alkyl, 4-Pyridyl-(C₁₋₄)-alkyl, 3-Pyridyl-(C₁₋₄)-alkyl, 2-Pyridyl-(C₁₋₄)-alkyl, Triazolyl-(C₁₋₄)-alkyl und 1-Imidazolyl-(C₁₋₄)-alkyl. Beispiele für Halogenatome sind F-, Cl-, oder Br-Atome, wobei Cl-Atome ganz besonders bevorzugt sind. Bevorzugte C₁-C₄₋Aminoalkylgruppen sind die Aminomethyl-, die Aminoethyl und die Aminopropylgruppe. Bevorzugte C₁-C₄-Acylgruppen sind Formyl, Acetyl, Propionyl und Butyryl. Eine bevorzugte C₁₋₄-Carboxyalkylgruppe ist die 3-Carboxypropylgruppe. Beispiele für eine Aryl-C₁-C₄-alkylgruppe sind Benzyl und 2-Phenylethyl. Besonders bevorzugte C₂-C₆-Alkenylengruppen sind Vinylen und Propylen. Eine besonders bevorzugte C₄-C₆-Alkadienylengruppe ist die 1,3-Butadien-1,4-diylgruppe. Unter funktionellen Carbonylderivaten werden Kondensationsprodukte aus Carbonylverbindunen und Verbindungen mit einer NH₂-Funktionalität verstanden. Beispiele für funktionelle Carbonylderivate sind Oxime und Imine. Die weiteren verwendeten Begriffe leiten sich erfindungsgemäß von den hier gegebenen Definitionen ab.

Die Verbindungen mit den Formeln Ia, Ib und II sind zum großen Teil literaturbekannt, im Handel erhältlich oder nach bekannten Syntheseverfahren herstellbar.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel la sind aromatische oder heteroaromatische Aldehyde oder Ketone, wie
- 5-(4'-Dimethylaminophenyl)-penta-2,4-dienal, 5-(4'-Diethylaminophenyl)-penta-2,4-dienal, 5-(4'-Methoxyphenyl)-penta-2,4-dienal, 5-(3',4'-Dimethoxyphenyl)-penta-2,4-dienal, 5-(2',4'-Dimethoxyphenyl)-penta-2,4-dienal, 5-(4'-Piperidinophenyl)-penta-2,4-dienal, 5-(4'-Morpholinophenyl)-penta-2,4-dienal, 5-(4'-Pyrrolidinophenyl)-penta-2,4-dienal, 6-(4'-Dimethylaminophenyl)-hexa-2,4-dien-2-on, 6-(4'-Diethylaminophenyl)-hexa-2,4-dien-2-on, 6-(4'-Methoxyphenyl)-hexa-2,4-dien-2-on, 6-(3',4'-Dimethoxyphenyl)-hexa-2,4-dien-2-on, 6-(2',4'-Dimethoxyphenyl)-hexa-2,4-dien-2-on, 6-(4'-Piperidinophenyl)-hexa-2,4-dien-2-on, 6-(4'-Morpholinophenyl)-hexa-2,4-dien-2-on, 6-(4'-Pyrrolidinophenyl)-hexa-2,4-dien-2-on, 5-(4'-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4'-Acetylphenyl)-trimethylammonium-, 4-(N,N-Diethyl)-N-methylammonio)-benzaldehyd-, N-(4'-Benzoylphenyl)-trimethylammonium-, N-(4'-Benzoylphenyl)-N,N-diethylmethylammonium-, N-(4'-Formylphenyl)-N-methylpyrrolidinium-, N-(4'-Formylphenyl)-N-methylpiperidinium-, N-(4'-Formylphenyl)-N-methylmorpholinium-, N-(4'-Acetylphenyl)-N-methylmorpholinium-, N-(4'-Benzoylphenyl)-N-methylmorpholinium-, 3-Formyl-N-ethyl-N-methylcarbazolium-, 3-Formyl-9,9-dimethylcarbazolium-, 1-(4'-Acetylphenyl)-3-methylimidazolium-, 1-(4'-Acetylphenyl)-3-methyl-2-imidazolinium-, 1-(4'-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorozinkate, Methylsulfate, Trifluorrnethansulfonate, Hexafluorophosphate, Tetrafluoroborate,
- Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-l-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolinium-, 1,3-Dimethyl-2-(4'-formylphenyl)-imidazolinium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3'-Formylphenyl)-3-methylbenzothiazolium-, 2-(4'-Formylnaphth-1-yl)-3-methylbenzothiazolium-, 5-Chlor-2-(4'-formylphenyl)-3-methylbenzothiazolium-, 2-(4'-Formylphenyl)-3,5-dimethylbenzothiazolium-, 1-Methyl-2-[2-(4'-formylphenyl)-ethenyl]-pyridinium-, 1-Methyl-4-[2-(4'-acetylphenyl)-ethenyl]-pyridinium-, 1-Benzyl-4-[2-(4'-formylphenyl)-ethenyl]-pyridinium, 1-Methyl-4-[2-(4'-formylphenyl)-ethenyl]-pyridinium-, 1-Methyl-2-[2-(4'-formylphenyl)-ethenyl]-pyridinium-, 1-Methyl-4-[2-(4'-formylphenyl)-ethenyl]-chinolinium, 1-Methyl-2-[2-(4'-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4'-formylphenyl)-ethenyl]-benzothiazolinium-, 1,3-Dimethyl-2-[2-(4'-formylphenyl)-ethenyl]-benzimidazolinium-, 1,3-Dimethyl-2-[2-(4'-formylphenyl)-ethenyl]-imidazolinium-, 1-Methyl-5-oxo-indeno[1,2-b]pyridinium(4-methyl-4-azonio-9-fluorenon-), 1-Ethyl-5-oxo-indeno[1,2-b]pyridinium(4-Ethyl-4-azonio-9-fluorenon-), 1-Benzyl-5-oxo-indeno[1,2-b]pyridinium(-4-benzyl-4-azonio-9-fluorenon-), 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-salze, insbesondere Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylaminozimtaldehyd, 4-(Dibutylamino)-benzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1 H,5H-benzo[ij]chinolizin-9-carboxaldehyd, 4-(1-lmidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoylacetophenon, 2-(4'-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibromsalicil, 2,2'-Furyl, 2,2'-Thienyl, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-, -Dimethyl-, -Dibrom-, -Dichlor-, -Bis-dimethylamino-, 2,4-Dihydroxy-, 3,3'-Dimethoxy, 2'-Chlor-3,4-dimethoxy-, 3,4,5,3',4',5'-Hexamethoxybenzil,
- Isatinderivate, wie 5-Chlorisatin, 5-Methoxyisatin, 5-Nitroisatin, 6-Nitroisatin, 5-Sulfoisatin, lsatin-5-sulfonsäure, lsatin-4-carbonsäure und lsatin-5-carbonsäure,
- N-substituierte Isatin-Derivate, wie N-Methylisatin, N-(2'-Hydroxyalkyl)-isatin, N-(2'-Hydroxypropyl)-isatin, N-(3'-Hydroxypropyl)-isatin, N-(2',3'-Dihydroxypropyl)-isatin, N-(2'-Sulfoethyl)-isatin, (3'-Sulfopropyl)-isatin, N-Allylisatin, N-Vinylisatin, N-Benzylisatin, N-(4'-Methoxybenzyl)-isatin, N-(4'-Carboxybenzyl)-isatin, N-(4'-Sulfobenzyl)-isatin, N-(2'-Dimethylaminoethyl)-isatin, N-(2'-Pyrrolidinoethyl)-isatin, N-(2'-Piperidinoethyl)-isatin, (2'-Morpholinoethyl)-isatin, N-(2'-Furylmethyl)-isatin, N-(Thien-2-ylmethyl)-isatin, N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2'-hydroxyethyl)-isatin, 5-Methyl-N-(2'-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2'-hydroxyethyl)-aminomethyl)-isatin, N-(2'-Hydroxyethylaminomethyl)-isatin, N-(Bis-(2'-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3'-Carboxypropylaminomethyl)-isatin, N-(Bis-(2'-hydroxyethyl)-aminomethyl)-5-methylisatin, N-Piperidinomethyl-5-chlorisatin, N-(2'-Sulfoethylamino)-isatin, sowie die Alkali- und gegebenenfalls Ammoniumsalze der sauren Verbindungen,
   Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxy-acetophenon, 4-Amino-acetophenon, 4-Dimethylaminoacetophenon, 4-Morpholino-acetophenon, 4-Piperidino-acetophenon, 4-Imidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, lmidazol-2-aldehyd,
- Indanon-Derivate, wie z. B. 1,2-Indandion, 2-Oximo-1-indanon, Indan-1,2,3-trion-2-oxim, 5-Methoxy-indan-1,2,3-trion-2-oxim, 2-Nitro-1,3-indandion
sowie beliebige Gemische der voranstehenden Verbindungen.

Bevorzugte Verbindungen mit der Formel Ib sind Maleindialdehyd, Fumardialdehyd, Orthoameisensäuretrimethylester, Orthoameisensäuretriethylester, Glyoxal, Malondialdehyd, Malcndialdehyd-bis-dimethylacetal, Malondialdehyd-bis-diethylacetal, Glutaconaldehyd sowie die Metall- und Ammoniumsalze der Dialdehyde.

Vorzugsweise sind die Verbindungen mit der Formel II ausgewählt aus 1-Acetyl-indolin-3-on, 1-Propionyl-indolin-3-on, 1-Acetyl-5-chlor-indolin-3-on, 1-Acetyl-5-brom-indolin-3-on, 1-Acetyl-5-nitro-indolin-3-on, 1-Acetyl-5-carboxy-indolin-3-on, 1-Acetyl-5-ethoxycarbonyl-indolin-3-on, Acetyl-5-methoxycarbonyl-indolin-3-on, 1-Acetyl-5-methyl-indolin-3-on, 1-Acetyl-5-methoxy-indolin-3-on, 1-Acetyl-3-acetoxyindol, sowie ihren hautverträglichen Säureadditionsprodukten.

Die voranstehend genannten Verbindungen mit der Formel I oder II werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.
Unter die voranstehend beschriebene Ausführungsform fällt ebenfalls die Verwendung von solchen Substanzen, die Reaktionsprodukte der Aldehyde oder Ketone mit den Formeln la und/oder lb und 1-Acylindolin-3-onen mit der Formel II als direktziehende Färbemittel. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

In allen Färbemitteln können auch mehrere verschiedene Verbindungen mit den Formeln Ia und Ib und der Formel II gemeinsam zum Einsatz kommen.

Färbungen mit noch erhöhter Brillanz und verbesserten Echtheitseigenschaften (Lichtechtheit, Waschechtheit, Reibechtheit) über einen weiten Nuancenbereich werden erzielt, wenn die erfindungsgemäß eingesetzten Verbindungen der Formeln Ia bzw. Ib und II gemeinsam mit mindestens einer weiteren Komponente (im folgenden Komponente C genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und deren physiologisch verträglichen Salzen, sowie anderen CH-aciden Verbindungen und quartären Ammoniumverbindungen verwendet werden. Dies sind einerseits Verbindungen, die für sich alleine keratinhaltige Fasern nur schwach färben und erst gemeinsam mit den Verbindungen der Formeln la bzw. lb und II brillante Färbungen ergeben. Andererseits sind darunter aber auch Verbindungen, die bereits als Oxidationsfarbstoffvorprodukte eingesetzt werden.

Geeignete Verbindungen mit primärer oder sekundärer Aminogruppe als Komponente C sind z. B. primäre und sekundäre aromatische Amine wie N,N-Dimethyl-, N,N-Diethyl-, N-(2'-Hydroxyethyl)-N-ethyl-, N,N-Bis-(2'-hydroxyethyl)-, N-(2'-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2',5'-Diaminophenyl)-ethanol, 2,4-Diaminophenoxyethanol, 2-(2',5'-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2'-hydroxyethylamino)-, 6-Methyl-3-amino-2-chlor-, 2-Methyl-5-amino-4-chlor-, 3,4-Methylendioxy-, 5-(2'-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, Bis-(5'-amino-2'-hydroxyphenyl)-methan, aromatische Nitrile, Nitrogruppen-haltige Aminoverbindungen, wie 3-Amino-6-methylamino-2-nitropyridin, Pikraminsäure, [8-[(4'-Amino-2'-nitrophenyl)azo]-7-hydroxy-naphth-2-yl]-trimethylammoniumchlorid, [8-((4'-Amino-3'-nitrophenyl)azo)-7-hydroxy-naphth-2-yl]-trimethylammoniumchlorid (Basic Brown 17), 1-Hydroxy-2-amino-4,6-dinitrobenzol, 1-Amino-2-nitro-4-bis-(2'-hydroxyethyl)-amino-benzol, 1-Amino-2-(2'-hydroxyethyl)-amino-5-nitrobenzol (HC Yellow Nr. 5), 1-Amino-2-nitro-4-(2'-hydroxyethyl)-aminobenzol (HC Red Nr. 7), 2-Chloro-5-nitro-N-hydroxyethyl-1,4-phenylendiamin, 1-(2'-Hydroxyethyl)-amino-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2'-hydroxyethyl)-amino-6-nitrobenzol (HC Violet Nr. 1), 1-Amino-2-nitro-4-(2',3'-dihydroxypropyl)-amino-5-chlorobenzol (HC Red Nr. 10), 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure Dinatriumsalz (Acid blue Nr. 29), 1-Amino-2-(2'-hydroxy-4'-nitrophenylazo)-8-naphthol-3,6-disulfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-suffonsäure, 4'-Amino-3'-nitrobenzophenon-2-carbonsäure, 1-Amino-4-nitro-2-(2-nitrobenzylidenamino)-benzol, 2-[2'-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2'-hydroxyethylamino)-benzol, 2-Nitro-1-amino-4-[bis-(2'-hydroxyethyl)-amino]-benzol, 4-Amino-2-nitrodiphenylamin-2'-carbonsäure, 1-Amino-5-chlor-4-(2'-hydroyethylamino)-2-nitrobenzol, aromatische Aniline bzw. Phenole mit einem weiteren aromatischen Rest, wie sie in der Formel IV dargestellt sind 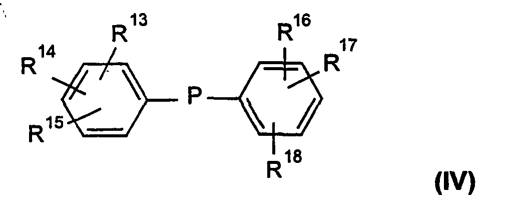 in der
- R¹³ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R¹⁴, R¹⁵, R¹⁶, R¹⁷ und R¹⁸ unabhängig voneinander für ein Wasserstoffatom, eine Hydroxy- oder eine Aminogruppe, die durch C₁₋₄-Alkyl-, C₁₋₄₋Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel V

   **Q'-(CH**_{**2**}**-Q-CH**_{**2**}**-Q")**_{**o**} **(V)**

   in der
- Q eine direkte Bindung, eine CH₂- oder CHOH-Gruppe bedeutet,
- Q' und Q" unabhängig voneinander für ein Sauerstoffatom, eine NR¹⁹⁻Gruppe, worin R¹⁹ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
- o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodipheny-tetrahydrochlorid, 3,3',4,4'-Tetraamino-benzophenon, 1,3-Bis-(2',4'-diaminophenoxy)-propan-tetrahydrochlorid, 1,8-Bis-(2',5'-diaminophenoxy)-3,6-dioxaoctan-tetrahydrochlorid, 1,3-Bis-(4'-aminophenylamino)propan, -2-propanol, 1,3-Bis-[N-(4'-aminophenyl)-2-hydroxyethylamino]-2-propanol, N,N-Bis-[2-(4'-aminophenoxy)-ethyl]-methylamin-trihydrochlorid, N-Phenyl-1,4-phenylendiamin, 1,4-Bis-(4'-aminophenyl)-1,4-diazacycloheptan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 1-Phenyl-, 1-(2'-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2'-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Prolin, Lysin und Tryptophan. Aber auch andere Aminosäuren, wie z.B. 6-Aminocapronsäure und β-Alanin, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Als CH-acide Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Diiminoisoindolin und 2-Amino-4-imino-1,3-thiazolin-hydrochlorid.

Beispiele für quartäre Ammoniumsalze sind Tetramethyl-, Tetraethyl-, Tetrapropyl-, Tetrabutyl-, Benzyltrimethylammonium-chlorid, -bromid, -iodid, - hydrogensulfat, -(halb)sulfat sowie Polyquaternium 10.

Die Verbindungen der Komponente C werden besonders bevorzugt ausgewählt aus der Gruppe bestehend aus N-(2'-Hydroxyethyl)-N-ethyl-, 2-Chlor-p-phenylendiamin, N,N-Bis-(2'-hydroxyethyl)-p-phenylendiamin, 4-Aminophenol, 2-Amino-6-chloro-4-nitrophenol, p-Phenylendiamin, 2-(2',5'-Diaminophenyl)-ethanol, 2,5-Diaminotoluol, 3,4-Methylendioxyanilin, 2-Amino-4-(2'-hydroxyethylamino)-anisol, 2-(2',4'-Diaminophenoxy)-ethanol, 3-Amino-2,4-dichlor-, 2-Methyl-5-amino-, 3-Methyl-4-amino-, 2-Methyl-5-(2'-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, Bis-(4,5-amino-2-hydroxyphenyl)-methan, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2'-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), β-Alanin, L-Prolin, L-Lysin, DL-Tyrosin sowie deren vorzugsweise mit anorganischen Säuren gebildeten physiologisch verträglichen Salze.

Die voranstehend genannten Verbindungen der Komponente C können in einer Menge von jeweils 0,03 bis 65 mmol, insbesondere 1 bis 40 mmol, jeweils bezogen auf 100 g des gesamten Färbemittels, eingesetzt werden.

In einer weiteren Ausführungsform kann die Bandbreite des Nuancenbereichs der Ausfärbungen durch die Verwendung von reaktiven Carbonylverbindungen als zusätzliche Komponente D erweitert werden. Beispiele für geeignete reaktive Carbonylverbindungen sind Aldehyde oder cyclische oder nichtcyclische Ketone. Diese Verbindungen können mit den Aminoverbindungen bzw. CH-aciden Verbindungen der Komponente C reagieren. Durch geeignete Kombination der Verbindungen der Formeln la bzw. lb und II, und ggf. der Komponente C läßt sich eine große Vielfalt an Ausfärbungen erhalten. Unter diese Ausführungsform fällt auch die Verwendung von solchen Substanzen, die Reaktionsprodukte von Aldehyden und/oder Ketonen der Formeln Ia bzw. Ib und II mit den genannten Verbindungen der Komponente C und Verbindungen der Komponente D darstellen, als direktziehende Färbemittel.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel- mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ₋SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈₋Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S. 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/sobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung einer Kombination aus
**A**. mindestens einem Aldehyd und/oder einem Keton, ausgewählt aus Verbindungen der Formeln la und lb, und/oder deren funktionelle Carbonylderivate und/oder physiologisch verträgliche Salze davon, 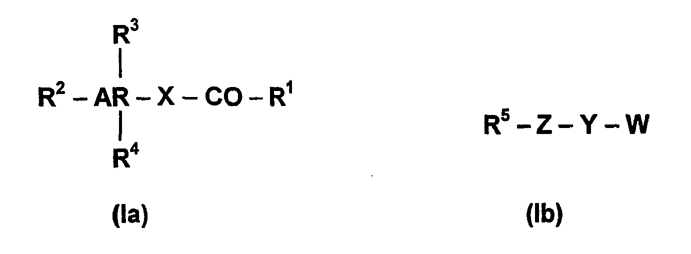 in der AR, R¹, R², R³ R⁴, X, R⁵, R⁶, W, Y und Z wie oben definiert sind, und
**B**. 1-Acylindolin-3-onen der Formel II oder einem Derivat oder physiologisch verträglichen Salzen davon, 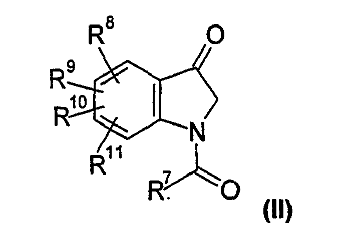 in der R⁷, R⁸, R⁹, R¹⁰ und R¹¹ wie oben definiert sind,
als färbende Komponenten in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
**A**. mindestens einem Aldehyd und/oder einem Keton, ausgewählt aus Verbindungen der Formeln la und lb, und/oder deren funktionelle Carbonylderivate und/oder physiologisch verträgliche Salze davon, 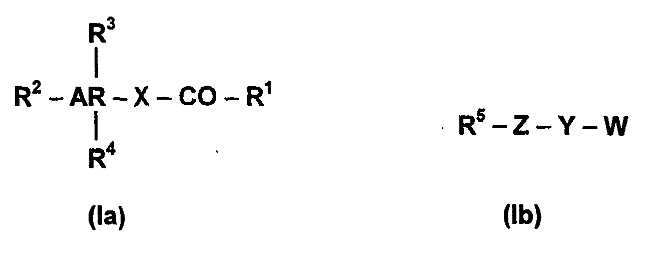 in der AR, R¹, R², R³ R⁴, X, R⁵, R⁶, W, Y und Z wie oben definiert sind, und
**B**. 1-Acylindolin-3-onen der Formel II oder einem Derivat oder physiologisch verträglichen Salzen davon, 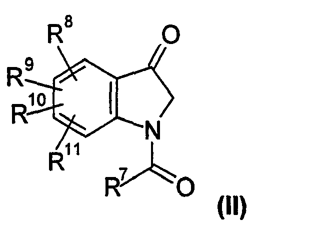 in der R⁷, R⁸, R⁹, R¹⁰ und R¹¹ wie oben definiert sind und gegebenenfalls
**C**. mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, aromatischen Hydroxyverbindungen, Aminosäuren, aus 2 bis 9 Aminosäuren aufgebauten Oligopeptiden und/oder mindestens eine CH-acide Verbindung oder quartäre Ammoniumverbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Kombination aus Aldehyden und/oder Ketonen der Formel la bzw. lb und 1-Acylindolin-3-onen der Formel II und die Verbindungen der Komponente C können entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel la bzw. Ib oder II oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Die Aldehyde und/oder Ketone mit der Formel Ia bzw. Ib, die 1-Acylindolin-3-one mit der Formel II und die Verbindungen der Komponente C können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiele

### Herstellung einer Färbelösung

Es wurde je eine Aufschlämmung von 5 mmol eines Aldehyds oder Ketons der Formel Ia bzw. Ib, 5 mmol einer 1-Acylindolin-3-ons mit der Formel II in 25 ml Wasser bei 50°C hergestellt. Die Aufschlämmungen wurden nach Abkühlen auf 30°C miteinander vermischt, mit 5 mmol Natriumacetat und einem Tropfen einer 25-%igen Fettalkylethersulfat-Lösung versetzt und mit verdünnter NaOH oder Salzsäure der pH-Wert gemäß Tabelle 1 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft (30°C bis 40°C) getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **Ausfärbungen mit einer Kombination aus Aldehyden und/oder Ketonen und 1-Acylindolin-3-onen** | | | | |
|---|---|---|---|---|
| **Aldehyd oder Keton der Formel Ia bzw. Ib** | **1-Acylindolin-3-on der Formel II** | **pH-Wert** | **Farbe** | **Int.** |
| 4-Formyl-1-methylchinolinium-p-toluolsulfonat | 1-Acetylindolin-3-on | 6,0 | rotviolett | ++ |
| 4-Formyl-1-methylchinolinium-p-toluolsulfonat | 1-Acetyl-3-acetoxyindol | 9,0 | violettrot | +++ |
| 4-Formyl-1-methylpyridinium-benzolsulfonat | 1-Acetylindolin-3-on | 6,0 | weinrot | ++(+) |
| 4-Formyl-1-methylpyridinium-benzolsulfonat | 1-Acetylindolin-3-on | 9,0 | orangebrau n | ++(+) |
| Coniferylaldehyd | 1-Acetylindolin-3-on | 6,0 | gelborange | ++ |
| Vanillin | 1-Acetylindolin-3-on | 6,0 | hellgelb | + |
| Isatin | 1-Acetylindolin-3-on | 6,0 | orange | ++(+) |
| 2-Dihydroxymethyl-1-methylchinolinium-p-toluolsulfonat | 1-Acetyl-3-acetoxyindol | 9,0 | blauviolett | ++(+) |
| 2-Dihydroxymethyl-1-methylchinolinium-p-toluolsulfonat | 1-Acetyl-3-indolinon | 9,0 | grauviolett | ++ |

## Patentansprüche

1. Mittel zum Färben von keratinhaltigen Fasern, insbesondere menschlichen Haaren, enthaltend
**A**. mindestens einen Aldehyd und/oder ein Keton, ausgewählt aus Verbindungen der Formeln la und lb, und/oder deren funktionelle Carbonylderivate und/oder physiologisch verträgliche Salze davon, 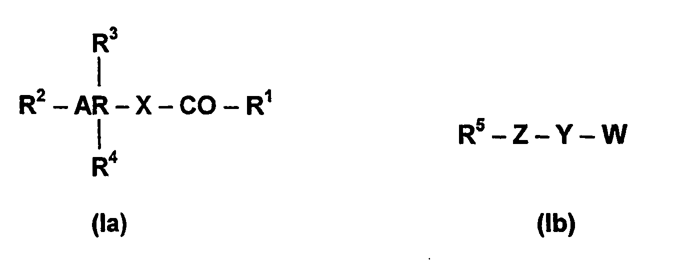 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₄-Acyl-, C₂-C₄₋Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁₋C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄₋alkylgruppe, eine Hydroxy-, Nitro-, Pyrrolidino, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₁-C₄₋Hydroxyalkoxygruppen, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, wobei auch zwei der Reste -X-CO-R¹, R², R³ und R⁴ zusammen einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, Methylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
• R⁵ steht für ein Wasserstoffatom oder eine C₁-C₄-Alkylgruppe,
• Z steht für eine Gruppe 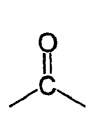 oder eine Gruppe 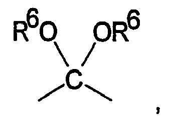 in der R⁶ eine C₁-C₄-Alkylgruppe bedeutet,
• Y steht für eine direkte Bindung, eine C₁-C₄-Alkylengruppe, eine ggf. substituierte C₂-C₄-Alkenylen- oder C₄-C₆-Alkadienylengruppe oder eine Gruppe CHOR^{6a}, in der R^{6a} ein Wasserstoffatom oder eine C₁-C₄₋Alkylgruppe bedeutet,
• W steht für ein Wasserstoffatom, eine C₁-C₄-Alkoxygruppe, eine Formylgruppe oder eine Gruppe 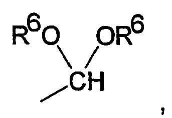 in der R⁶ eine C₁-C₄₋Alkylgruppe bedeutet,
**B**. mindestens ein 1-Acylindolin-3-on mit der Formel II oder ein Derivat davon oder deren physiologisch verträglichen Salze, 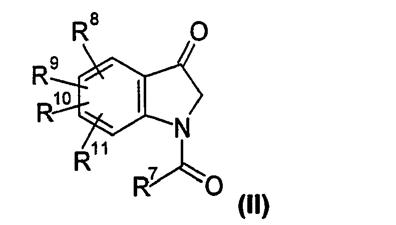 wobei
• R⁷ steht für ein Wasserstoffatom, eine C₁-C₄-Alkyl-, eine Aryl-, eine Aryl-C₁-C₄-alkyl- oder eine Heteroarylgruppe und
• R⁸, R⁹, R¹⁰ und R¹¹ stehen jeweils unabhängig voneinander für ein Wasserstoffatom, ein Halogenatom, eine C₁-C₄-Alkyl-, C₁-C₄-Alkoxy- oder C₁-C₄-Hydroxyalkoxygruppe, eine Hydroxy-, Nitro-, Carboxy-, Carboxylato-, C₁-C₄-Alkoxycarbonyl-, eine gegebenenfalls substituierte Carbamoyl-, Sulfo-, Sulfato-, Sulfamoyl- oder Aminogruppe, die durch C₁-C₄-Alkyl- oder C₁-C₄-Hydroxyalkylgruppen substituiert sein kann.

2. Mittel nach Anspruch 1, **dadurch gekennzeichnet, dass** die Verbindungen mit der Formel la ausgewählt sind aus aromatischen und/oder heteroaromatischen Aldehyden und/oder Ketonen, insbesondere aus
- 5-(4'-Dimethylaminophenyl)-penta-2,4-dienal, 5-(4'-Diethylaminophenyl)-penta-2,4-dienal, 5-(4'-Methoxyphenyl)-penta-2,4-dienal, 5-(3',4'-Dimethoxyphenyl)-penta-2,4-dienal, 5-(2',4'-Dimethoxyphenyl)-penta-2,4-dienal, 5-(4'-Piperidinophenyl)-penta-2,4-dienal, 5-(4'-Morpholinophenyl)-penta-2,4-dienal, 5-(4'-Pyrrolidinophenyl)-penta-2,4-dienal, 6-(4'-Dimethylaminophenyl)-hexa-2,4-dien-2-on, 6-(4'-Diethylaminophenyl)-hexa-2,4-dien-2-on, 6-(4'-Methoxyphenyl)-hexa-2,4-dien-2-on, 6-(3',4'-Dimethoxyphenyl)-hexa-2,4-dien-2-on, 6-(2',4'-Dimethoxyphenyl)-hexa-2,4-dien-2-on, 6-(4'-Piperidinophenyl)-hexa-2,4-dien-2-on, 6-(4'-Morpholinophenyl)-hexa-2,4-dien-2-on, 6-(4'-Pyrrolidinophenyl)-hexa-2,4-dien-2-on, 5-(4'-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4'-Acetylphenyl)-trimethylammonium-, 4-(N,N-Diethyl)-N-methylammonio)-benzaldehyd-, N-(4'-Benzoylphenyl)-trimethylammonium-, N-(4'-Benzoylphenyl)-N,N-diethylmethylammonium-, N-(4'-Formylphenyl)-N-methylpyrrolidinium-, N-(4'-Formylphenyl)-N-methylpiperidinium-, N-(4'-Formylphenyl)-N-methylmorpholinium-, N-(4'-Acetylphenyl)-N-methylmorpholinium-, N-(4'-Benzoylphenyl)-N-methylmorpholinium-, 3-Formyl-N-ethyl-N-methylcarbazolium-, 3-Formyl-9,9-dimethylcarbazolium-, 1-(4'-Acetylphenyl)-3-methylimidazolium-, 1-(4'-Acetylphenyl)-3-methyl-2-imidazolinium-, 1-(4'-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorozinkate, Methylsulfate, Trifluormethansulfonate, Hexafluorophosphate, Tetrafluoroborate,
- Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinoliniurn-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolinium-, 1,3-Dimethyl-2-(4'-formylphenyl)-imidazolinium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3'-Formylphenyl)-3-methylbenzothiazolium-, 2-(4'-Formylnaphth-1-yl)-3-methylbenzothiazolium-, 5-Chlor-2-(4'-formylphenyl)-3-methylbenzothiazolium-, 2-(4'-Formylphenyl)-3,5-dimethylbenzothiazolium-, 1-Methyl-2-[2-(4'-formylphenyl)-ethenyl]-pyridinium-, 1-Methyl-4-[2-(4'-acetylphenyl)-ethenyl]-pyridinium-, 1-Benzyl-4-[2-(4'-formylphenyl)-ethenyl]-pyridinium, 1-Methyl-4-[2-(4'-formylphenyl)-ethenyl]-pyridinium-, 1-Methyl-2-[2-(4'-formylphenyl)-ethenyl]-pyridinium-, 1-Methyl-4-[2-(4'-formylphenyl)-ethenyl]-chinolinium, 1-Methyl-2-[2-(4'-formylphenyl)-ethenyl]-chinolinium-, 1-Methyl-2-[2-(5-formyl-2-furyl)-ethenyl]-chinolinium-, 1 Methyl-2-[2-(5-formy)-2-thienyl)-ethenyl]-chinolinium-, 1-Methyl-2-[2-(4'-formylphenyl)-ethenyl]-benzothiazolinium-, 1,3-Dimethyl-2-[2-(4'-formylphenyl)-ethenyl]-benzimidazolinium-, 1,3-Dimethyl-2-[2-(4'-formylphenyl)-ethenyl]-imidazolinium-, 1-Methyl-5-oxo-indeno[1,2-b]pyridinium(4-methyl-4-azonio-9-fluorenon-), 1-Ethyl-5-oxo-indeno[1,2-b]pyridinium(4-Ethyl-4-azonio-9-fluorenon-), 1-Benzyl-5-oxo-indeno[1,2-b]pyridinium(-4-benzyl-4-azonio-9-fluorenon-), 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-salze, insbesondere Benzolsulfonat, p-Toluolsulfonat, Methansulfonat, Perchlorat, Sulfat, Chlorid, Bromid, lodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylaminozimtaldehyd, 4-(Dibutylamino)-benzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibrornsalicil, 2,2'-Furyl, 2,2'-Thienyl, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-, -Dimethyl-, -Dibrom-, -Dichlor-, -Bis-dimethylamino-, 2,4-Dihydroxy-, 3,3'-Dimethoxy, 2'-Chlor-3,4-dimethoxy-, 3,4,5,3',4',5'-Hexamethoxybenzil,
- Isatinderivate, wie 5-Chlorisatin, 5-Methoxyisatin, 5-Nitroisatin, 6-Nitroisatin, 5-Sulfoisatin, Isatin-5-sulfonsäure, Isatin-4-carbonsäure und Isatin-5-carbonsäure,
- N-substituierte Isatin-Derivate, wie N-Methylisatin, N-(2'-Hydroxyalkyl)-isatin, N-(2'-Hydroxypropyl)-isatin, N-(3'-Hydroxypropyl)-isatin, N-(2',3'-Dihydroxypropyl)-isatin, N-(2'-Sulfoethyl)-isatin, (3'-Sulfopropyl)-isatin, N-Allylisatin, N-Vinylisatin, N-Benzylisatin, N-(4'-Methoxybenzyl)-isatin, N-(4'-Carboxybenzyl)-isatin, N-(4'-Sulfobenzyl)-isatin, N-(2'-Dimethylaminoethyl)-isatin, N-(2'-Pyrrolidinoethyl)-isatin, N-(2'-Piperidinoethyl)-isatin, (2'-Morpholinoethyl)-isatin, N-(2'-Furylmethyl)-isatin, N-(Thien-2-ylmethyl)-isatin, N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2'-hydroxyethyl)-isatin, 5-Methyl-N-(2'-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2'hdroethyl)-aminomethyl)-isatin, N-(2'-Hydroxyethylaminomethyl)-isatin, N-(Bis-(2'-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3'-Carboxypropylaminomethyl)-isatin, N-(Bis-(2'-hydroxyethyl)-aminomethyl)-5-methylisatin, N-Piperidinomethyl-5-chlorisatin, N-(2'-Sulfoethylamino)-isatin, sowie die Alkali- und gegebenenfalls Ammoniumsalze der sauren Verbindungen,
Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxy-acetophenon, 4-Amino-acetophenon, 4-Dimethylamino-acetophenon, 4-Morpholino-acetophenon, 4-Piperidino-acetophenon, 4-Imidazolino-acetophenon, 2-Hydroxy-5-bromacetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd,
- Indanon-Derivate, wie z. B. 1,2-Indandion, 2-Oximo-1-indanon, Indan-1,2,3-trion-2-oxim, 5-Methoxy-indan-1,2,3-trion-2-oxim, 2-Nitro-1,3-indandion
sowie beliebigen Gemischen der voranstehenden Verbindungen.

3. Mittel nach Anspruch 1, **dadurch gekennzeichnet, dass** die Verbindungen mit der Formel Ib ausgewählt sind aus Maleindialdehyd, Fumardialdehyd, Orthoameisensäuretrimethylester, Orthoameisensäuretriethylester, Glyoxal, Malondialdehyd, Malondialdehyd-bis-dimethylacetal, Malondialdehyd-bis-diethylacetal, Glutaconaldehyd sowie den Metall- und Ammoniumsalzen der Dialdehyde.

4. Mittel nach einem der Ansprüche 1 bis 3, **dadurch gekennzeichnet, dass** die Verbindungen mit der Formel II ausgewählt sind aus 1-Acetylindolin-3-on, 1-Propionyl-indolin-3-on, 1-Acetyl-5-chlor-indolin-3-on, 1-Acetyl-5-brom-indolin-3-on, 1-Acetyl-5-nitro-indolin-3-on, 1-Acetyl-5-carboxy-indolin-3-on, 1-Acetyl-5-ethoxycarbonyl-indolin-3-on, Acetyl-5-methoxycarbonyl-indolin-3-on, 1-Acetyl-5-methyl-indolin-3-on, 1-Acetyl-5-methoxy-indolin-3-on, 1-Acetyl-3-acetoxyindol ihren hautverträglichen Säureadditionsprodukten und beliebigen Gemischen der voranstehenden Verbindungen.

5. Mittel nach einem der Ansprüche 1 bis 4, **dadurch gekennzeichnet, dass** die Aldehyde und/oder Ketone der Formel la und/oder lb und die 1-Acylindolin-3-one der Formel II jeweils in einer Menge von 0,03 bis 65 mmol, insbesondere von 1 bis 40 mmol, bezogen auf 100 g des gesamten Färbemittels, enthalten sind.

6. Mittel nach einem der Ansprüche 1 bis 5, **dadurch gekennzeichnet, dass** es zusätzlich mindestens eine Verbindung C mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens eine CH-acide Verbindung und/oder quartäre Ammoniumverbindung enthält.

7. Mittel nach Anspruch 6, **dadurch gekennzeichnet, dass** die Verbindung C ausgewählt ist aus primären und sekundären aromatischen Aminen wie N,N-Dimethyl-, N,N-Diethyl-, N-(2'-Hydroxyethyl)-N-ethyl-, N,N-Bis-(2'-hydroxyethyl)-, N-(2'-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, - phenol, -phenethol, 4-Amino-3-methylphenol, 2-(2',5'-Diaminophenyl)-ethanol, 2,4-Diaminophenoxyethanol, 2-(2',5'-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2'-hydroxyethylamino)-, 6-Methyl-3-amino-2-chlor-, 2-Methyl-5-amino-4-chlor-, 3,4-Methylendioxy-, 5-(2'-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, Bis-(5'-amino-2'-hydroxyphenyl)-methan, aromatische Nitrile, Nitrogruppen-haltige Aminoverbindungen, wie 3-Amino-6-methylamino-2-nitro-pyridin, Pikraminsäure, [8-[(4'-Amino-2'-nitrophenyl)azo]-7-hydroxynaphth-2-yl]-trimethylammoniumchlorid, [8-((4'-Amino-3'-nitrophenyl)azo)-7-hydroxy-naphth-2-yl]-trimethylammoniumchlorid (Basic Brown 17), 1-Hydroxy-2-amino-4,6-dinitrobenzol, 1-Amino-2-nitro-4-bis-(2'-hydroxyethyl)-aminobenzol, 1-Amino-2-(2'-hydroxyethyl)-amino-5-nitrobenzol (HC Yellow Nr. 5), 1-Amino-2-nitro-4-(2'-hydroxyethyl)-aminobenzol (HC Red Nr. 7), 2-Chloro-5-nitro-N-hydroxyethyl-1,4-phenylendiamin, 1-(2'-Hydroxyethyl)-amino-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2'-hydroxyethyl)-amino-6-nitrobenzol (HC Violet Nr. 1), 1-Amino-2-nitro-4-(2',3'-dihydroxypropyl)-amino-5-chlorobenzol (HC Red Nr. 10), 2-(4'-Amino-2'-nitroanilino)-benzoesäure, 6-Nitro-2,5-diaminopyridin, 2-Amino-6-chloro-4-nitrophenol, 1-Amino-2-(3'-nitrophenylazo)-7-phenylazo-8-naphthol-3,6-disulfonsäure Dinatriumsalz (Acid blue Nr. 29), 1-Amino-2-(2'-hydroxy-4'-nitrophenylazo)-8-naphthol-3,6-disulfonsäure Dinatriumsalz (Palatinchrome green), 1-Amino-2-(3'-chlor-2'-hydroxy-5'-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2'-(Diethylamino)ethylamino]-5-nitroanilin, 3-Amino-4-hydroxy-5-nitrobenzolsulfonsäure, 3-Amino-3'-nitrobiphenyl, 3-Amino-4-nitroacenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2'-hydroxyethylamino)-benzol, 2-Nitro-1-amino-4-[bis-(2'-hydroxyethyl)-amino]-benzol, 4-Amino-2-nitrodiphenylamin-2'-carbonsäure, 1-Amino-5-chlor-4-(2'-hydroyethylamino)-2-nitrobenzol, aromatischen Anilinen. bzw. Phenolen mit einem weiteren aromatischen Rest, wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2',4'-diaminophenoxy)-propan, 1,8-Bis-(2',5'-diaminophenoxy)-3,6-dioxaoctan, 1,3-Bis-(4'-aminophenylamino)propan, -2-propanol, 1,3-Bis-[N-(4'-aminophenyl)-2-hydroxyethylamino]-2-propanol, N,N-Bis-[2-(4'-aminophenoxy)-ethyl]-methylamin-trihydrochlorid, N-Phenyl-1,4-phenylendiamin, 1,4-Bis-(4'-aminophenyl)-1,4-diazacycloheptan,
stickstoffhaltige heterocyclische Verbindungen ausgewählt aus der Gruppe bestehend aus 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 1-Phenyl-, 1-(2'-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin, Hydroxypyrimidin-Derivate, sowie aromatischen Hydroxyverbindungen wie 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2'-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure,
Aminosäuren ausgewählt aus der Gruppe Arginin, Histidin, Tyrosin, Phenylalanin, DOPA (Dihydroxyphenylalanin), Ornithin, Prolin, Lysin, Tryptophan, 6-Aminocapronsäure und β-Alanin,
Oligopeptiden ausgewählt aus Glutathion oder die in den Hydrolysaten von Kollagen, Keratin, Casein, Elastin, Sojaprotein, Weizengluten oder Mandelprotein enthaltenen Oligopeptide,
CH-aciden Verbindungen ausgewählt aus der Gruppe 1,2,3,3-Tetramethyl-3H-indoliumiodid, 1,2,3,3-Tetramethyl-3H-indofium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluoisulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Cyanmethylenindan, 1,3-Diiminoisoindolin und 2-Amino-4-imino-1,3-thiazolin-hydrochlorid,
quartären Ammoniumsalzen ausgewählt aus der Gruppe Tetramethyl-, Tetraethyl-, Tetrapropyl-, Tetrabutyl-, Benzyltrimethylammonium-chlorid, - bromid, -iodid, -hydrogensulfat, -(halb)sulfat sowie Polyquaternium 10.

8. Mittel nach Anspruch 7, **dadurch gekennzeichnet, dass** die weitere Verbindung ausgewählt ist aus der Gruppe bestehend aus N-(2'-Hydroxyethyl)-N-ethyl-, 2-Chlor-p-phenylendiamin, N,N-Bis-(2'-hydroxyethyl)-p-phenylendiamin, 4-Aminophenol, 2-Amino-6-chloro-4-nitrophenol, p-Phenylendiamin, 2-(2',5'-Diaminophenyl)-ethanol, 2,5-Diaminotoluol, 3,4-Methylendioxyanilin, 2-Amino-4-(2'-hydroxyethylamino)-anisol, 2-(2',4'-Diaminophenoxy)-ethanol, 3-Amino-2,4-dichlor-, 2-Methyl-5-amino-, 3-Methyl-4-amino-, 2-Methyl-5-(2'-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2'-hydroxyethyl)-pyrazol, 5,6-Dihydroxyindol und 5,6-Dihydroxyindolin, Tetrabutylammoniumbromid, β-Alanin, L-Prolin, L-Lysin, DL-Tyrosin sowie jeweils aus den vorzugsweise mit anorganischen Säuren gebildeten physiologisch verträglichen Salzen dieser Verbindungen.

9. Mittel nach einem der Ansprüche 1 bis 8, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

10. Mittel nach einem der Ansprüche 1 bis 9, **dadurch gekennzeichnet, dass** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

11. Mittel nach einem der Ansprüche 1 bis 10, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, enthalten sind.

12. Mittel nach einem der Ansprüche 1 bis 11, **dadurch gekennzeichnet, dass** es Oxidationsmittel, insbesondere H₂O₂, in einer Menge von 0,01 bis 6 Gew.%, bezogen auf die Anwendungslösung, enthält.

13. Mittel nach einem der Ansprüche 1 bis 12, **dadurch gekennzeichnet, dass** es anionische, zwitterionische oder nichtionische Tenside enthält.

14. Verwendung einer Kombination aus
A. mindestens einem Aldehyd und/oder einem Keton, ausgewählt aus Verbindungen mit den Formeln la und/oder Ib, und/oder deren funktionelle Carbonylderivate und/oder physiologisch verträgliche Salze davon, 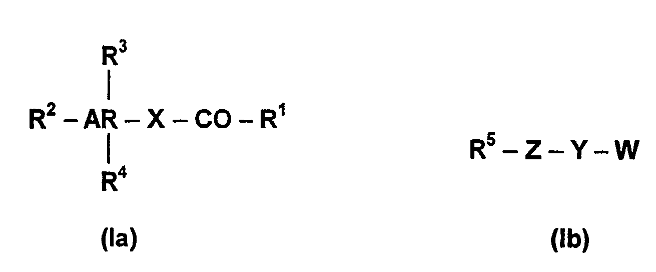 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₄-Acyl-, C₂-C₄₋Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁₋C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄₋alkylgruppe, eine Hydroxy-, Nitro-, Pyrrolidino, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₁₋C₄-Hydroxyalkoxygruppen, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, wobei auch zwei der Reste ―X-CO-R¹, R², R³ und R⁴ zusammen einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, Methylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
• R⁵ steht für ein Wasserstoffatom oder eine C₁-C₄-Alkylgruppe,
• Z steht für eine Gruppe 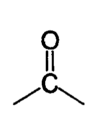 oder eine Gruppe 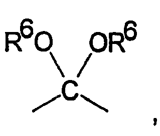 in der R⁶ eine C₁-C₄-Alkylgruppe bedeutet,
• Y steht für eine direkte Bindung, eine C₁-C₄₇-Alkylengruppe, eine ggf. substituierte C₂-C₄-Alkenylen- oder C₄-C₆-Alkadienylengruppe oder eine Gruppe CHOR^{6a}, in der R^{6a} ein Wasserstoffatom oder eine C₁-C₄₋Alkylgruppe bedeutet,
• W steht für ein Wasserstoffatom, eine C₁-C₄-Alkoxygruppe, eine Formylgruppe oder eine Gruppe 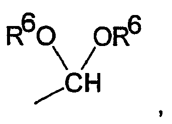 in der R⁶ eine C₁-C₄₋Alkylgruppe bedeutet,
**B**. mindestens einem 1-Acylindolin-3-on mit der Formel II oder ein Derivat davon oder deren physiologisch verträglichen Salze, 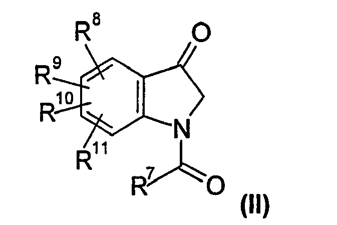 wobei
• R⁷ steht für ein Wasserstoffatom, eine C₁-C₄-Alkyl-, eine Aryl-, eine Aryl-C₁-C₄-alkyl- oder eine Heteroarylgruppe und
• R⁸, R⁹, R¹⁰ und R¹¹ stehen jeweils unabhängig voneinander für ein Wasserstoffatom, ein Halogenatom, eine C₁-C₄-Alkyl-, C₁-C₄-Alkoxy- oder C₁-C₄-Hydroxyalkoxygruppe, eine Hydroxy-, Nitro-, Carboxy-, Carboxylato-, C₁-C₄-Alkoxycarbonyl-, eine gegebenenfalls substituierte Carbamoyl-, Sulfo-, Sulfato-, Sulfamoyl- oder Aminogruppe, die durch C₁-C₄-Alkyl- oder C₁-C₄-Hydroxyalkylgruppen substituiert sein kann,
als eine färbende Komponente in Oxidationshaarfärbemitteln.

15. Verfahren zum Färben von keratinhaltigen Fasern, insbesondere menschlichen Haaren, worin ein Färbemittel, enthaltend
**A.** mindestens einen Aldehyd und/oder ein Keton, ausgewählt aus Verbindungen mit den Formeln Ia und Ib, und/oder deren funktionelle Carbonylderivate und/oder physiologisch verträgliche Salze davon, 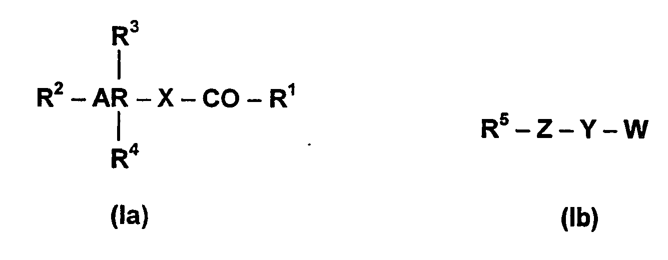 wobei
• AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R¹ steht für ein Wasserstoffatom, eine C₁-C₄-Alkyl-, C₁-C₄-Acyl-, C₂-C₄₋Alkenyl-, C₁-C₄-Perfluoralkyl-, eine ggf. substituierte Aryl- oder Heteroarylgruppe,
• R², R³ und R⁴ stehen unabhängig voneinander für ein Wasserstoffatom, ein Halogenatom, eine C₁-C₄-Alkyl-, C₁₋₄-Alkoxy-, C₁-C₄-Aminoalkyl-, C₁₋C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄₋alkylgruppe, eine Hydroxy-, Nitro-, Pyrrolidino, Morpholino-, Piperidino-, Amino- bzw. Ammonio- oder 1-Imidazol(in)iogruppe, wobei die letzten drei Gruppen mit C₁₋₄-Alkyl-, C₁₋₄-Carboxyalkyl-, C₁₋₄-Hydroxyalkyl-, C₁₋C₄-Hydroxyalkoxygruppen, C₂₋₄-Alkenylgruppen, mit ggf. substituierten Benzylgruppen, mit Sulfo-(C₁₋₄)-alkyl- oder Heterozyklus-(C₁₋₄)-alkylgruppen substituiert sein können, wobei auch zwei der Reste ―X-CO-R¹, R², R³ und R⁴ zusammen einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R², R³ und R⁴,
• X steht für eine direkte Bindung, eine Carbonyl-, Methylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann,
• R⁵ steht für ein Wasserstoffatom oder eine C₁-C₄-Alkylgruppe,
• Z steht für eine Gruppe 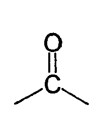 oder eine Gruppe 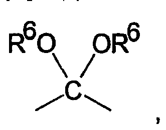 in der R⁶ eine C₁-C₄-Alkylgruppe bedeutet,
• Y steht für eine direkte Bindung, eine C₁-C₄-Alkylengruppe, eine ggf. substituierte C₂-C₄-Alkenylen- oder C₄-C₆-Alkadienylengruppe oder eine Gruppe CHOR^{6a}, in der R^{6a} ein Wasserstoffatom oder eine C₁-C₄₋Alkylgruppe bedeutet,
• W steht für ein Wasserstoffatom, eine C₁-C₄-Alkoxygruppe, eine Formylgruppe oder eine Gruppe 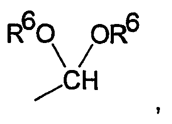 in der R⁶ eine C₁-C₄₋Alkylgruppe bedeutet,
**B**. mindestens ein 1-Acylindolin-3-on mit der Formel II oder ein Derivat davon oder deren physiologisch verträglichen Salze, 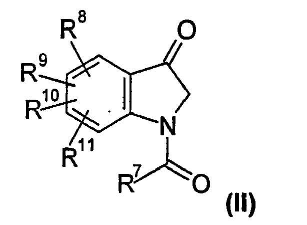 wobei
• R⁷ steht für ein Wasserstoffatom, eine C₁-C₄-Alkyl-, eine Aryl-, eine Aryl-C₁-C₄-alkyl- oder eine Heteroarylgruppe und
• R⁸, R⁹, R¹⁰ und R¹¹ stehen jeweils unabhängig voneinander für ein Wasserstoffatom, ein Halogenatom, eine C₁-C₄-Alkyl-, C₁-C₄-Alkoxy- oder C₁-C₄-Hydroxyalkoxygruppe, eine Hydroxy-, Nitro-, Carboxy-, Carboxylato-, C₁-C₄-Alkoxycarbonyl-, eine gegebenenfalls substituierte Carbamoyl-, Sulfo-, Sulfato-, Sulfamoyl- oder Aminogruppe, die durch C₁-C₄-Alkyl- oder C₁-C₄-Hydroxyalkylgruppen substituiert sein kann,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

16. Verfahren gemäß Anspruch 15, **dadurch gekennzeichnet, daß** neben Komponente A und Komponente B eine weitere Komponente
**C**. bestehend aus mindestens einer Verbindung mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder mindestens einer CH-aciden Verbindung oder quartären Ammoniumverbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for colouring fibres containing keratin, in particular human hair, comprising
A. at least one aldehyde and/or one ketone chosen from the compounds of the formulae Ia and Ib, and/or their functional carbonyl derivatives and/or physiologically compatible salts thereof, 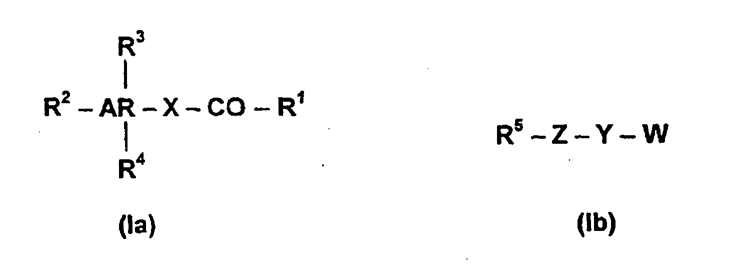 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁₋C₄-acyl group, C₂-C₄-alkenyl group, C₁-C₄₋perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄₋hydroxyalkyl groups, C₁-C₄-hydroxyalkoxy groups, C₂₋₄-alkenyl groups, by optionally substituted benzyl groups, by sulpho-(C₁₋₄)-alkyl groups or heterocycle-(C₁₋₄)-alkyl groups, where two of the radicals -X-CO-R¹, R², R³ and R⁴ can together form a fused-on optionally substituted 5-, 6- or 7-membered ring, which may likewise carry a fused-on aromatic ring, where the system AR can, depending on the size of the ring, carry further substituents, which may, independently of one another, be the same groups as R², R³ and R⁴,
• X is a direct bond, a carbonyl group, methylene group, an optionally substituted C₂-C₆-alkylene group, C₄-C₆-alkadienylene group, furylene group, thienylene group, arylene, group, vinylenearylene group, vinylenefurylene group, vinylenethienylene group, where X, together with the CO-R¹ group can also form an optionally substituted 5-,6- or 7-membered ring,
• R⁵ is a hydrogen atom or a C₁-C₄-alkyl group
• Z is a group 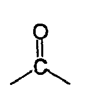 or a group 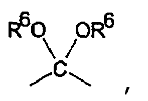 in which R⁶ is a C₁-C₄-alkyl group,
• Y is a direct bond, a C₁-C₄-alkylene group, an optionally substituted C₂-C₄-alkenylene group or C₄-C₆-alkadienylene group or a group CHOR^{6a}, in which R^{6a} is a hydrogen atom or a C₁-C₄-alkyl group,
• W is a hydrogen atom, a C₁-C₄-alkoxy group, a formyl group or a group 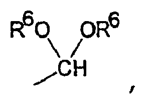
in which R⁶ is a C₁-C₄-alkyl group,
**B**. at least one 1-acylinodolin-3-one with the formula II or a derivative thereof or physiologically compatible salts thereof, 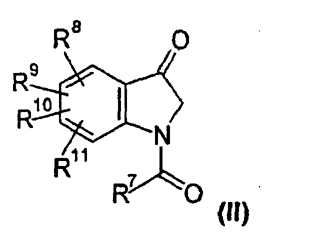 where
• R⁷ is a hydrogen atom, a C₁-C₄-alkyl group, an aryl group, an aryl-C₁-C₄-alkyl group or a heteroaryl group and
• R⁸, R⁹, R¹⁰ and R¹¹, in each case independently of one another, are a hydrogen atom, a halogen atom, a C₁-C₄-alkyl group, C₁-C₄-alkoxy group or C₁-C₄-hydroxyalkoxy group, a hydroxy group, nitro group, carboxy group, carboxylato group, C₁-C₄-alkoxycarbonyl group, an optionally substituted carbamoyl group, sulpho group, sulphato group, sulphamoyl group or amino group, which may be substituted by C₁-C₄-alkyl or C₁-C₄-hydroxyalkyl groups.

2. Agent according to Claim 1, **characterized in that** the compounds with the formulae Ia are chosen from aromatic and/or heteroaromatic aldehydes and/or ketones, in particular from
- 5-(4'-dimethylaminophenyl)penta-2,4-dienal, 5-(4'-diethylaminophenyl)penta-2,4-dienal, 5-(4'-methoxyphenyl)penta-2,4-dienal, 5-(3',4'-dimethoxyphenyl)penta-2,4-dienal, 5-(2',4'-dimethoxyphenyl)penta-2,4-dienal 5(4'-piperidinophenyl)penta-2,4-dienal 5-(4'-morpholinophenyl)penta-2,4-dienal, 5-(4'-pyrrolidinophenyl)penta-2,4-dienal, 6-(4'-dimethylaminophenyl)hexa-2,4-dien-2-one, 6-(4'-diethylaminophenyl)hexa-2,4-dien-2-one, 6-(4'-methoxyphenyl)hexa-2,4-dien-2-one, 6-(3',4'-dimethoxyphenyl)hexa-2,4-dien-2-one, 6-(2',4'-dimethoxyphenyl)hexa-2,4-dien-2-one, 6-(4'-piperidinophenyl)hexa-2,4-dien-2-one, 6-(4'-morpholinophenyl)hexa-2,4-dien-2-one, 6-(4'-pyrrolidinophenyl)hexa-2,4-dien-2-one, 5-(4'-dimethylaminonaphth-1-yl)penta-2,4-dienal,
- 2-nitropiperonal, 5-nitropiperonal, 6-nitropiperonal, 5-hydroxy-2-nitropiperonal, 2-hydroxy-5-nitropiperonal, 2-chloro-6-nitropiperonal, 5-chloro-2-nitropiperonal, 2,6-dinitropiperonal,
- 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 4-hydroxy-3-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 5-nitrovanillin, 3,5-dinitrosalicylaldehyde, 5-bromo-3-nitrosalicylaldehyde, 3-nitro-4-formylbenzenesulphonic acid, 4-nitro-l-naphthaldehyde, 2-nitrocinnamaldehyde, 3-nitrocinnamaldehyde, 4-nitrocinnamaldehyde,
- carbazole aldehydes or carbazole ketones, in particular 9-methyl-3-carbazole aldehyde, 9-ethyl-3-carbazole aldehyde, 3-aceylcarbazole, 3,6-diacetyl-3-ethylcarbazole, 3-acetyl-9-methylcarbazole, 1,4-dimethyl-3-carbazole aldehyde, 1,4,9-trimethyl-3-carbazole aldehyde,
- salts of 4-trimethylammoniobenzaldehyde, 4-benzyldimethylammoniobenzaldehyde, 4-trimethylammoniocinnamaldehyde, 4-trimethylammonionaphthaldehyde, 2-methoxy-4-trimethylammoniobenzaldehyde, N-(4'-acetylphenyl)trimethylammonium, 4-((N,N-diethyl)-N-methylammonio)benzaldehyde, N-(4'-benzoylphenyl)trimethylammonium, N-(4'-benzoylphenyl)-N,N-diethylmethylammonium, N-(4'-formylphenyl)-N-methylpyrrolidinium, N-(4'-formylphenyl)-N-methylpiperidinium, N-(4'-formylphenyl-N-methylmorpholinium, N-(4'-acetylphenyl)-N-methylmorpholinium, N-(4'-benzoylphenyl)-N-methylmorpholinium, 3-formyl-N-ethyl-N-methylcarbazolium, 3-formyl-9,9-dimethylcarbazolium, 1-(4'-acetylphenyl)-3-methylimidazolium, 1-(4'-acetylphenyl)-3-methyl-2-imidazolinium, 1-(4'-benzoylphenyl)-3-methylimidazolium, 5-acetyl-1,3-diethyl-2-methylbenzimidazolium, 5-trimethylammonio-1-indanone, in particular the benzenesulphonates, p-toluenesulphonates, methanesulphonates, ethanesulphonates, propanesulphonates, perchlorates, sulphates, chlorides, bromides, iodides, tetrachlorozincates, methylsulphates, trifluoromethanesulphonates, hexafluorophosphates, tetrafluoroborates,
- salts of formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 4-acetyl-1-methylpyridinium, 2-acetyl-1-methylpyridinium, 4-acetyl-1-methylquinolinium, 5-formyl-1-methylquinolinium, 6-formyl-1-methylquinolinium, 7-formyl-1-methylquinolinium, 8-formyl-1-methylquinolinium, 5-formyl-1-ethylquinolinium, 6-formyl-1-ethylquinolinium, 7-formyl-1-ethylquinolinium, 8-formyl-1-ethylquinolinium, 5-formyl-1-benzylquinoliniium, 6-formyl-1-benzylquinolinium, 7-formyl-1-benzylquinolinium, 8-formyl-1-benzylquinolinium, 5-formyl-1-allylquinolinium, 6-formyl-1-allylquinolinium, 7-formyl-1-allylquinolinium and 8-formyl-1-allyquinolinium, 5-acetyl-1-methylquinolinium, 6-acetyl-1-methylquinolinium, 7-acetyl-1-methylquinolinium, 8-acetyl-1-methylquinolinium, 5-acetyl-1-ethylquinolinium, 6-acetyl-1-ethylquinolinium, 7-acetyl-1-ethylquinolinium, 8-acetyl-1-ethylquinolinium, 5-acetyl-1-benzylquinolinium, 6-acetyl-1-benzylquinolinium, 7-acetyl-1-benzylquinolinium, 8-acetyl-1-benzylquinolinium, 5-acetyl-1-allylquinolinium, 6-acetyl-1-allylquinolinium, 7-acetyl-1-allylquinolinium and 8-acetyl-1-allylquinolinium, 9-formyl-10-methylacridinium, 4-(2'-formylvinyl)-1-methylpyridinium, 1,3-dimethyl-2-(4'-formylphenyl)-benzimidazolinium, 1,3-dimethyl-2-(4'-formylphenyl)imidazolinium, 2-(4'-formylphenyl)-3-methylbenzothiazolium, 2-(4'-acetylphenyl)-3-methylbenzothiazolium, 2-(4'-formylphenyl)-3-methylbenzoxazolium, 2-(5-formyl-2-furyl)-3-methylbenzoxazolium, 2-(5-formyl-2-thienyl)-3-methylbenzoxazolium, 2-(3'-formylphenyl)-3-methylbenzothiazolium, 2-(4'-formylnaphth-1-yl)-3-methylbenzothiazolium, 5-chloro-2-(4'-formylphenyl)-3-methylbenzothiazolium, 2-(4'-formylphenyl)-3,5-dimethylbenzothiazolium, 1-methyl-2-[2-(4'-formylphenyl)ethenyl]-pyridinium, 1-methyl-4-[2-(4'-acetylphenyl)ethenyl]pyridinium, 1-benzyl-4-[2-(4'-formylphenyl)ethenyl]pyridinium, 1-methyl-4-[2-(4'-formylphenyl)ethenyl]pyridinium, 1-methyl-2-[2-(4'-formylphenylethenyl]pyridinium, 1-methyl-4-[2-(4'-formylphenyl)ethenyl]quinolinium, 1-methyl-2-[2-(4'-formylphenyl)ethenyl]quinolinium, 1-methyl-2-[2-(5-formyl-2-furyl)ethenyl]quinolinium, 1-methyl-2-[2-(5-formyl-2-thienyl)ethenyl]quinolinium, 1-methyl-2-[3-(4'-formylphenyl)-ethenyl]benzothiazolinium, 1,3-dimethyl-2-[2-(4'-formylphenyl)ethenyl]benzimidazolinium, 1,3-dimethyl-2-[2-(4'-formylphenyl)ethenyl]-imidazolinium, 1-methyl-5-oxoindeno[1,2-b]-pyridinium (4-methyl-4-azonio-9-fluorenone), 1-ethyl-5-oxoindeno[1,2-b]pyridinium(4-ethyl-4-azonio-9-fluorenone), 1-benzyl-5-oxoindeno[1,2-b]pyridinium(4-benzyl-4-azonio-9-fluorenone), 2-methyl-5-oxoindeno[1,2-c]-pyridinium, 2-methyl-9-oxoindeno[2,1-c]-pyridinium, 1-methyl-9-oxoindeno[2,1-b]-pyridinium, in particular benzenesulphonate, p-toluenesulphonate, methanesulphonate, perchlorate, sulphate, chloride, bromide, iodide, tetrachlorozincate, methylsulphate, trifluoromethanesulphonate, tetrafluoroborate,
- salicylaldehyde, vanillin, 4-hydroxy-3-methoxycinnamaldehyde (coniferyl aldehyde), 2,4-dihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 4-piperidinobenzaldehyde, 4-dimethylaminoacetophenone, 4-hydroxynaphthaldehyde, 4-dimethylaminonaphthaldehyde, 4-dimethylaminobenzylideneacetone, 4-dimethylaminocinnamaldehyde, 2-dimethylaminobenzaldehyde, 2-chloro-4-dimethylaminobenzaldehyde, 4-dimethylamino-2-methylbenzaldehyde, trans-4-diethylaminocinnamaldehyde, 4-(dibutylamino)benzaldehyde, 4-diphenylaminobenzaldehyde, 2,3,6,7-tetrahydro-1H,5H-benzo[ij]quinolizine-9-carboxaldehyde, 4-dimethylamino-2-methoxybenzaldehyde, 2,3,6,7-tetrahydro-8-hydroxy-1H,5H-benzo[ij]quinolizine-9-carboxaldehyde, 4-(1-imidazolylbenzaldehyde, 2-morpholinobenzaldehyde, indole-3-carboxaldehyde, 1-methylindole-3-carboxaldehyde, N-ethylcarbazole-3-carboxaldehyde, 2-formylmethylene-1,3,3-trimethylindoline (tribase aldehyde)
- 1,3-diacetylbenzene, 1,4-diacetylbenzene, 1,3,5-triacetylbenzene, 2-benzoylacetophenone, 2-(4'-methoxybenzoyl)acetophenone, 2-(2-furoyl)acetophenone, 2-(2-pyridoyl)-acetophenone, 2-(3-pyridoyl)acetophenone,
- 1-phenyl-1,2-propanedione, 1-phenyl-1,2-butanedione, 1-(chlorophenyl)-1,2-propanedione, 1-phenyl-3,3-dimethyl-1,2-butanedione, benzil, anisil, salicil, 5,5'-dibromosalicil, 2,2'-furyl, 2,2'-thienyl, 2,2'-, 4,4'-pyridil, 6,6'-dimethyl-4,4'-pyridil, 4-hydroxy-, 4-methoxy-, 4-chloro-, 4-methyl-, 4-dimethylamino-, 4,4'-dihydroxy-, -dimethyl-, -dibromo-, -dichloro-, -bisdimethylamino-, 2,4-dihydroxy-, 3,3'-dimethoxy, 2'-chloro-3,4-dimethoxy-, 3,4,5,3',4',5'-hexamethoxybenzil,
- isatin derivatives, such as 5-chloroisatin, 5-methoxyisatin, 5-nitroisatin, 6-nitroisatin, 5-sulphoisatin, isatin-5-sulphonic acid, isatin-4-carboxylic acid and isatin-5-carboxylic acid,
- N-substituted isatin derivatives, such as N-methylisatin, N-(2'-hydroxyalkyl)isatin, N-(2'-hydroxypropyl)isatin, N-(3'-hydroxypropyl)isatin, N-(2',3'-dihydroxypropyl)isatin, N-(2'-sulphoethyl)isatin, (3'-sulphopropyl)-isatin, N-allylisatin, N-vinylisatin, N-benzylisatin, N-(4'-methoxybenzyl)isatin, N-(4'-carboxybenzyl)isatin, N-(4'-sulphobenzyl)isatin, N-(2'-dimethylaminoethyl)isatin, N-(2'-pyrrolidinoethyl)isatin, N-(2'-piperinoethyl)isatin, (2'-morpholinoethyl)isatin, N-(2'-furylmethyl)isatin, N-(thien-2-ylmethyl)isatin, N-(pyrid-2-ylmethyl)isatin, N-(pyrid-3-ylmethyl)isatin, N-(pyrid-4-ylmethyl)-isatin, N-allylisatin-5-sulphonic acid, 5-chloro-N-(2'-hydroxyethyl)isatin, 5-methyl-N-(2'-hydroxyethyl)isatin, 5,7-dichloro-N-allylisatin, 5-nitro-N-allylsatin, N-hydroxymethylisatin, N-hydroxymethyl-5-methylisatin, N-hydroxymethyl-5-chloroisatin, N-hydroxymethyl-5-sulphoisatin, N-hydroxymethyl-5-carboxyisatin, N-hydroxymethyl-5-nitroisatin, N-hydroxymethyl-5-bromoisatin, N-hydroxymethyl-5-methoxyisatin, N-hydroxymethyl-5,7-dichloroisatin, N-dimethylaminomethylisatin, N-diethylaminomethylisatin, N-(bis(2'-hydroxyethyl)aminomethyl)isatin, N-(2'-hydroxyethylaminomethyl)isatin, N-(bis(2'-hydroxypropyl)aminomethyl)isatin, N-pyrrolidinomethylisatin, N-piperidinomethylisatin, N-morpholinomethylisatin, N-(1,2,4-triazolyl)methylisatin, N-(1-imidazolyl)-methylisatin, N-carboxymethylaminomethylisatin, N-(2-carboxyethylaminomethyl)isatin, N-(3'-carboxypropylaminomethyl)isatin, N-(bis(2'-hydroxymethyl)aminomethyl)-5-methylisatin, N-piperidinomethyl-5-chloroisatin, N-(2'-sulphoethylamino)isatin, and the alkali metal and optionally ammonium salts of the acidic compounds,
quinisatin and derivatives thereof, such as N-methylquinisatin,
- acetophenone, propiophenone, 2-hydroxyacetophenone, 3-hydroxyacetophenone, 4-hydroxyacetophenone, 2-hydroxypropiophenone, 3-hydroxypropiophenone, 4-hydroxypropiophenone, 2-hydroxybutyrophenone, 3-hydroxybutyrophenone, 4-hydroxybutyrophenone, 2,4-dihydroxyacetophenone, 2,5-dihydroxyacetophenone, 2,6-dihydroxyacetophenone, 2,3,4-trihydroxyacetophenone, 3,4,5-trihydroxyacetophenone, 2,4,6-trihydroxyacetophenone, 2,4,6-trimethoxyacetophenone, 3,4,5-trimethoxyacetophenone, 3,4,5-trimethoxyacetophenone diethyl ketal, 4-hydroxy-3-methoxyacetophenone, 3,5-dimethoxy-4-hydroxyacetophenone, 4-aminoacetophenone, 4-dimethylaminoacetophenone, 4-morpholinoacetophenone, 4-piperidinoacetophenone, 4-imidazolinoacetophenone, 2-hydroxy-5-bromoacetophenone, 4-hydroxy-3-nitroacetophenone, acetophenone-2-carboxylic acid, acetophenone-4-carboxylic acid, benzophenone, 4-hydroxybenzophenone, 2-aminobenzophenone, 4,4'-dihydroxybenzophenone, 2,4-dihydroxybenzophenone, 2,4,4'-trihydroxybenzophenone, 2,3,4-trihydroxybenzophenone, 2-hydroxy-1-acetonaphthone, 1-hydroxy-2-acetonaphthone, chromone, chromone-2-carboxylic acid, flavone, 3-hydroxyflavone, 3,5,7-trihydroxyflavone, 4',5,7-trihydroxyflavone, 5,6,7-trihydroxyflavone, quercetin, indanone, 9-fluorenone, 3-hydroxyfluorenone, anthrone, 1,8-dihydroxyanthrone,
- heterocyclic carbonyl compounds, such as 2-indolealdehyde, 3-indolealdehyde, 1-methylindole-3-aldehyde, 2-methylindole-3-aldehyde, 1-acetylindole-3-aldehyde, 3-acetylindole, 1-methyl-3-acetylindole, 2-(1,3,3-trimethyl-2-indolinylidene)acetaldehyde, 1-methylpyrrole-2-aldehyde, 1-methyl-2-acetylpyrrole, 1-pyridinealdehyde, 2-pyridinealdehyde, 3-pyridinealdehyde, 4-acetylpyridine, 2-acetylpyridine, 3-acetylpyridine, pyridoxal, quinoline-3-aldehyde, quinoline-4-aldehyde, antipyrine-4-aldehyde, furfural, 5-nitrofurfural, 2-thenoyl-trifluoroacetone, chromone-3-aldehyde, 3-(5-nitro-2-furyl)acrolein, 3-(2-furyl)acrolein, imidazole-2-aldehyde,
- indanone derivatives, such as, for example, 1,2-indanedione, 2-oximo-1-indanone, indane-1,2,3-trione 2-oxime, 5-methoxyindane-1,2,3-trione 2-oxime, 2-nitro-1,3-indanedione
and any mixtures of the above compounds.

3. Agent according to Claim 1, **characterized in that** the compounds with the formula Ib are chosen from malealdehyde, fumaraldehyde, trimethyl orthoformate, triethyl orthoformate, glyoxal, malonaldehyde, malonaldehyde bis(dimethyl acetal), malonaldehyde bis(diethyl acetal), glutaconaldehyde, and the metal and ammonium salts of the dialdehydes.

4. Agent according to one of Claims 1 to 3, **characterized in that** the compounds with the formula II are chosen from 1-acetylindolin-3-one, 1-propionylindolin-3-one, 1-acetyl-5-chloroindolin-3-one, 1-acetyl-5-bromoindolin-3-one, 1-acetyl-5-nitroindolin-3-one, 1-acetyl-5-carboxyindolin-3-one, 1-acetyl-5-ethoxycarbonylindolin-3-one, acetyl-5-methoxycarbonylindolin-3-one, 1-acetyl-5-methylindolin-3-one, 1-acetyl-5-methoxyindolin-3-one, 1-acetyl-3-acetoxyindole, their skin-compatible acid addition products and any mixtures of the above compounds.

5. Agent according to one of Claims 1 to 4, **characterized in that** the aldehydes and/or ketones of the formula Ia and/or Ib and the 1-acylindolin-3-ones of the formula II are present in each case in an amount of from 0.03 to 65 mmol, in particular from 1 to 40 mmol, based on 100 g of the total colourant.

6. Agent according to one of Claims 1 to 5, **characterized in that** it additionally comprises at least one compound C with a primary or secondary amino group or hydroxy group chosen from primary or secondary aromatic amines, nitrogen-containing heterocyclic compounds and aromatic hydroxy compounds, amino acids, oligopeptides constructed from 2 to 9 amino acids and/or at least one CH-acidic compound and/or quaternary ammonium compound.

7. Agent according to Claim 6, **characterized in that** the compound C is chosen from primary and secondary aromatic amines, such as N,N-dimethyl-, N,N-diethyl-, N-(2'-hydroxyethyl)-N-ethyl-, N,N-bis(2-hydroxyethyl)-, N-(2'-methoxyethyl)-, 2,3-, 2,4-, 2,5-dichloro-p-phenylenediamine, 2-chloro-p-phenylenediamine, 2,5-dihydroxy-4-morpholinoaniline dihydrobromide, 2-, 3-, 4-aminophenol, 2-aminomethyl-4-aminophenol, 2-hydroxymethyl-4-aminophenol, o-, p-phenylenediamine, o-tolylenediamine, 2,5-diaminotoluene, -phenol, -phenethol, 4-amino-3-methylphenol, 2-(2',5'-diaminophenyl)ethanol, 2,4-diaminophenoxyethanol, 2-(2',5'-diaminophenoxy)ethanol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2'-hydroxyethylamino)-, 6-methyl-3-amino-2-chloro-, 2-methyl-5-amino-4-chloro-, 3,4-methylenedioxy-, 5-(2'-hydroxyethylamino)-4-methoxy-2-methyl-, 4-amino-2-hydroxymethylphenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulphonic acid, 3-amino-4-hydroxybenzenesulphonic acid, 4-amino-3-hydroxynapthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-, 1,2,4-triaminobenzene, 1,2,4,5-tetraminobenzene, 2,4,5-triaminophenol, pentaaminobenzene, hexaaminobenzene, 2,4,6-triaminoresorcinol, 9,5-diaminopyrocatechol, 4,6-diaminopyrogallol, 3,5-diamino-4-hydroxypyrocatechol, bis(5'-amino-2'-hydroxyphenyl)-methane, aromatic nitriles, amino compounds containing nitro groups, such as 3-amino-6-methylamino-2-nitropyridine, picramic acid, [8-[(4'-amino-2'-nitrophenyl)azo]-7-hydroxynaphth-2-yl]trimethylammonium chloride, [8-((4'-amino-3'-nitrophenyl]azo)-7-hydroxynaphth-2-yl]trimethylammonium chloride (Basic Brown 17), 1-hydroxy-2-amino-4,6-dinitrobenzene, 1-amino-2-nitro-4-bis-(2'-hydroxyethyl)aminobenzene, 1-amino-2-(2'-hydroxyethyl)amino-5-nitrobenzene (HC Yellow No. 5), 1-amino-2-nitro-4-(2'-hydroxyethyl)aminobenzene (HC Red No. 7), 2-chloro-5-nitro-N-hydroxyethyl-1,4-phenylenediamine, 1-(2'-hydroxyethyl)amino-2-nitro-4-aminobenzene (HC Red No. 3), 4-amino-3-nitrophenol, 4-amino-2-nitrophenol, 6-nitro-o-toluidine, 1-amino-3-methyl-4-(2'-hydroxyethyl)amino-6-nitrobenzene (HC Violet No. 1), 1-amino-2-nitro-4-(2,3'-dihydroxypropyl)amino-5-chlorobenzene (HC Red No. 10), 2-(4'-amino-2'-nitroanilino)benzoic acid, 6-nitro-2,5-diaminopyridine, 2-amino-6-chloro-4-nitrophenol, 1-amino-2-(3'-nitrophenylazo)-7-phenylazo-8-naphthol-3,6-disulphonic acid disodium salt (Acid Blue No. 29), 1-amino-2-(2'-hydroxy-4'-nitrophenylazo)-8-naphthol-3,6-disulphonic acid disodium salt (Palatinchrome Green), 1-amino-2-(3'-chloro-2'-hydroxy-5'-nitrophenylazo)-8-naphthol-3,6-disulphonic acid disodium salt (Gallion), 4-amino-4'-nitrostilbene-2,2'-disulphonic acid disodium salt, 2,4-diamino-3',5'-dinitro-2'-hydroxy-5-methylazobenzene (Mordant Brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)benzene, 2-[2'-(diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-1-nitronaphthalene, 5-amino-6-nitrobenzo-1,3-dioxole, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2'-hydroxyethylamino)benzene, 2-nitro-1-amino-4-[bis(2'-hydroxyethyl)amino]benzene, 4-amino-2-nitrodiphenylamine-2'-carboxylic acid, 1-amino-5-chloro-4-(2'-hydroxyethylamino)-2-nitrobenzene, aromatic anilines and phenols with a further aromatic radical, such as, for example, 4,4'-diaminostilbene and its hydrochloride, 4,4'-diaminostilbene-2,2'-disulphonic acid mono- or di-Na salt, 4-amino-4'-dimethylaminostilbene and its hydrochloride, 4,4'-diaminodiphenylmethane, 4,4'-diaminodiphenylmethane sulphide, 4,4'-diaminodiphenylmethane sulphoxide, 4,4 diaminodiphenylmethamine, 4,4'-diaminodiphenylamine-2-sulphonic acid, 4,4'-diaminobenzophenone, 4,4'-diaminobenzophenone diphenyl ether, 3,3',4,4'-tetraaminodiphenyl tetrahydrochloride, 3,3',4,4'-tetraaminobenzophenone, 1,3-bis-(2',4'-diaminophenoxy)propane, 1,8-bis(2',5'-diaminophenoxy)-3,6-dioxaoctane, 1,3-bis(4'-aminophenylamino)propane, -2-propanol, 1,3-bis[N-(4'-aminophenyl)-2-hydroxyethylamino]-2-propanol, N,N-bis[2-(4'-aminophenoxy)ethyl]methylamine trihydrochloride, N-phenyl-1,4-phenylenediamine, 1,4-bis(4'-aminophenyl)-1,4-diazacycloheptane, nitrogen-containing heterocyclic compounds chosen from the group consisting of 2-, 3-, 4-amino-, 2-amino-3-hydroxy-, 2,6-diamino-, 2,5-diamino-, 2,3-diamino-, 2-dimethylamino-5-amino-, 2-methylamino-3-amino-6-methoxy-, 2,3-diamino-6-methoxy-, 2,6-dimethoxy-3,5-diamino-, 2,4,5-triamino-, 2,6-dihydroxy-3,4-dimethypyridine, 2,4-dihydroxy-5,6-diamino-, 4,5,6-triamino-, 4-hydroxy-2,5,6-triamino-, 2-hydroxy-4,5,6-triamino-, 2,4,5,6-tetraamino-, 2-methylamino-4,5,6-triamino-, 2,4-, 4,5-diamino-, 2-amino-4-methoxy-6-methylpyrimidine, 3,5-diaminopyrazole, -1,2,4-triazole, 3-amino-, 3-amino-5-hydroxypyrazole, 1-phenyl-, 1-(2'-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrin), 2-, 3-, 8-aminoquinoline, 4-aminoquinaldine, 2-, 6-aminonicotinic acid, 6-aminoisoquinoline, 5-, 6-aminoindazole, 5-, 7-aminobenzimidazole, -benzothiazole, 2,5-dihydroxy-4-morpholinoaniline, and indole and indoline derivatives, such as 4-, 5-, 6-, 7-aminoindole, 5,6-dihydroxyindole, 5,6-dihydroxyindoline and 4-hydroxyindoline, hydroxypyrimidine derivatives, and aromatic hydroxy compounds, such as 2-, 4-, 5-methylresorcinol, 2,5-dimethylresorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2'-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid, 3,6-dihydroxy-2,7-naphthalenesulphonic acid, amino acids chosen from the group arginine, histidine, tyrosine, phenylalanine, DOPA (dihydroxyphenylalanine), ornithine, proline, lysine, tryptophan, 6-aminocaproic acid and β-alanine,
oligopeptides chosen from glutathione or the oligopeptides present in the hydrolysates of collagen, keratin, casein, elastin, soya protein, wheat gluten or almond protein,
CH-acidic compounds chosen from the group 1,2,3,3-tetramethyl-3H-indolium iodide, 1,2,3,3-tetramethyl-3H-indolium p-toluenesulphonate, 1,2,3,3-tetramethyl-2H-indolium methanesulphonate, 1,3,3-trimethyl-2-methyleneindoline (Fischer's base), 2,3-dimethylbenzothiazolium iodide, 2,3-dimethylbenzothiazolium p-toluenesulphonate, 1,2-dimethylnaphtho[1,2-d]thiazolium p-toluenesulphonate, 1-ethyl-2-methylnaphtho[1,2-d]thiazolium p-toluenesulphonate, rhodanin, rhodanin-3-acetic acid, 1-methyl-2-quinaldinium iodide, 1-ethyl-2-quinaldinium iodide, 1,4-dimethylquinolinium iodide, barbituric acid, thiobarbituric acid, 1,3-dimethylthiobarbituric acid, 1,3-diethylthiobarbituric acid, oxindole, 3-indoxyl acetate, 2-coumaranone, 5-hydroxycoumaranone, 6-hydroxycourmaranone, 1-methyl-3-phenyl-2-pyrazolinone, indane-1,3-dione, indan-1-one, benzoylacetonitrile, 1-cyanomethyleneindane, 1,3-diiminoisoindoline and 2-amino-4-imino-1,3-thiazoline hydrochloride, quaternary ammonium salts chosen from the group tetramethyl-, tetraethyl-, tetrapropyl-, tetrabutyl-, benzyltrimethylammonium chloride, bromide, iodide, hydrogensulphate, (half) sulphate, and polyquaternium 10.

8. Agent according to Claim 7, **characterized in that** the further compound is chosen from the group consisting of N-(2'-hydroxyethyl)-N-ethyl, 2-chloro-p-phenylenediamine, N,N-bis(2'-hydroxyethyl)-p-phenylenediamine, 4-aminophenol, 2-amino-6-chloro-4-nitrophenol, p-phenylenediamine, 2-(2',5'-diaminophenyl)ethanol, 2,5-diaminotoluene, 3,4-methylenedioxyaniline, 2-amino-4-(2'-hydroxyethylamino)anisole, 2-(2',4'-diaminophenoxy)-ethanol, 3-amino-2,4-dichloro-, 2-methyl-5-amino-, 3-methyl-4-amino-, 2-methyl-5-(2'-hydroxyethylamino)-, 2-methyl-5-amino-4-chloro-, 6-methyl-3-amino-2-chloro-, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-[diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 4,5-diamino-1-(2'-hydroxyethyl)pyrazole, 5,6-dihydroxyindole and 5,6-dihydroxyindoline, tetrabutylammonium bromide, β-alanine, L-proline, L-lysine, DL-tyrosine, and in each case of the physiologically compatible salts of these compounds preferably formed with inorganic acids.

9. Agent according to one of Claims 1 to 8, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

10. Agent according to one of the Claims 1 to 9, **characterized in that** it comprises direct dyes from the group of nitrophenylenediamines, nitroaminophenols, anthraquinones or indophenols, preferably in an amount of from 0.01 to 20% by weight, based on the total colourant.

11. Agent according to one of Claims 1 to 10, **characterized in that** ammonium or metal salts chosen from the group of formates, carbonates, halides, sulphates, butyrates, valerates, capronates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are present.

12. Agent according to one of Claims 1 to 11, **characterized in that** it comprises oxidizing agents, in particular H₂O₂, in an amount of from 0.01 to 6% by weight, based on the application solution.

13. Agent according to one of Claims 1 to 12, **characterized in that** it comprises anionic, zwitterionic or nonionic surfactants.

14. Use of a combination of
**A**. at least one aldehyde and/or one ketone chosen from the compounds of the formulae Ia and Ib, and/or their functional carbonyl derivatives and/or physiologically compatible salts thereof, 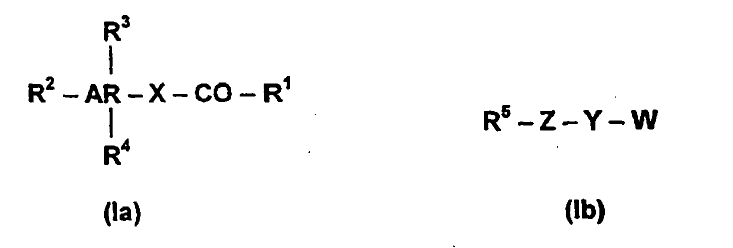 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁₋C₄-acyl group, C₂-C₄-alkenyl group, C₁-C₄₋perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄₋hydroxyalkyl groups, C₁-C₄-hydroxyalkoxy groups, C₂₋₄-alkenyl groups, by optionally substituted benzyl groups, by sulpho-(C₁₋₄)-alkyl groups or heterocycle-(C₁₋₄)-alkyl groups, where two of the radicals -X-CO-R¹, R², R³ and R⁴ can together form a fused-on optionally substituted 5-, 6- or 7-membered ring, which may likewise carry a fused-on aromatic ring, where the system AR can, depending on the size of the ring, carry further substituents, which may, independently of one another, be the same groups as R², R³ and R⁴,
• X is a direct bond, a carbonyl group, methylene group, an optionally substituted C₂-C₆-alkylene group, C₄-C₆-alkadienylene group, furylene group, thienylene group, arylene, group, vinylenearylene group, vinylenefurylene group, vinylenethienylene group, where X, together with the CO-R¹ group can also form an optionally substituted 5-,6- or 7-membered ring,
• R⁵ is a hydrogen atom or a C₁-C₄-alkyl group
• Z is a group 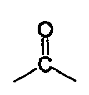 or a group 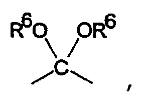 in which R⁶ is a C₁-C₄-alkyl group,
• Y is a direct bond, a C₁-C₄-alkylene group, an optionally substituted C₂-C₄-alkenylene group or C₄-C₆-alkadienylene group or a group CHOR^{6a}, in which R^{6a} is a hydrogen atom or a C₁-C₄-alkyl group,
• W is a hydrogen atom, a C₁-C₄-alkoxy group, a formyl group or a group 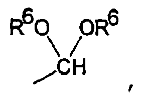
in which R⁶ is a C₁-C₄-alkyl group,
**B**. at least one 1-acylinodolin-3-one with the formula II or a derivative thereof or physiologically compatible salts thereof, 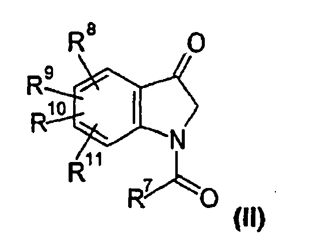 where
• R⁷ is a hydrogen atom, a C₁-C₄-alkyl group, an aryl group, an aryl-C₁-C₄-alkyl group or a heteroaryl group and
• R⁸, R⁹, R¹⁰ and R¹¹, in each case independently of one another, are a hydrogen atom, a halogen atom, a C₁-C₄-alkyl group, C₁-C₄-alkoxy group or C₁-C₄-hydroxyalkoxy group, a hydroxy group, nitro group, carboxy group, carboxylato group, C₁-C₄-alkoxycarbonyl group, an optionally substituted carbamoyl group, sulpho group, sulphato group, sulphamoyl group or amino group, which may be substituted by C₁-C₄-alkyl or C₁-C₄-hydroxyalkyl groups, as a colouring component in oxidation hair colourants.

15. Method of colouring fibres containing keratin, in particular human hair, in which a colourant comprising
**A**. at least one aldehyde and/or one ketone chosen from the compounds of the formulae Ia and Ib, and/or their functional carbonyl derivatives and/or physiologically compatible salts thereof, 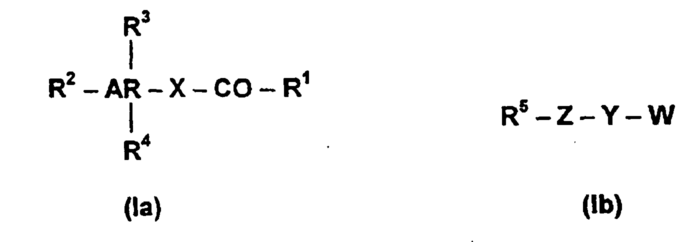 where
• AR is benzene, naphthalene, pyridine, pyrimidine, pyrazine, pyrazidine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R¹ is a hydrogen atom, a C₁-C₄-alkyl group, C₁₋C₄-acyl group, C₂-C₄-alkenyl group, C₁-C₄₋perfluoroalkyl group, an optionally substituted aryl group or heteroaryl group,
• R², R³ and R⁴, independently of one another, are a hydrogen atom, a halogen atom, a C₁-C₄-alkyl group, C₁₋₄-alkoxy group, C₁-C₄-aminoalkyl group, C₁-C₄-hydroxyalkyl group, a C₁-C₄-acyl group, a carboxy group, carboxylato group, sulpho group, sulphato group, C₂-C₆-alkenyl group, aryl group, an aryl-C₁-C₄-alkyl group, a hydroxy group, nitro group, pyrrolidino group, morpholino group, piperidino group, amino group or ammonio group or 1-imidazol(in)io group, where the last three groups may be substituted by C₁₋₄-alkyl groups, C₁₋₄-carboxyalkyl groups, C₁₋₄₋hydroxyalkyl groups, C₁-C₄-hydroxyalkoxy groups, C₂₋₄-alkenyl groups, by optionally substituted benzyl groups, by sulpho-(C₁₋₄)-alkyl groups or heterocycle-(C₁₋₄)-alkyl groups, where two of the radicals -X-CO-R¹, R², R³ and R⁴ can together form a fused-on optionally substituted 5-, 6- or 7-membered ring, which may likewise carry a fused-on aromatic ring, where the system AR can, depending on the size of the ring, carry further substituents, which may, independently of one another, be the same groups as R², R³ and R⁴,
• X is a direct bond, a carbonyl group, methylene group, an optionally substituted C₂-C₆-alkylene group, C₄-C₆-alkadienylene group, furylene group, thienylene group, arylene, group, vinylenearylene group, vinylenefurylene group, vinylenethienylene group, where X, together with the CO-R¹ group can also form an optionally substituted 5-,6- or 7-membered ring,
• R⁵ is a hydrogen atom or a C₁-C₄-alkyl group
• Z is a group 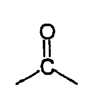 or a group 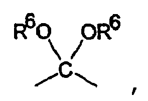 in which R⁶ is a C₁-C₄-alkyl group,
• Y is a direct bond, a C₁-C₄-alkylene group, an optionally substituted C₂-C₄-alkenylene group or C₄-C₆-alkadienylene group or a group CHOR^{6a}, in which R^{6a} is a hydrogen atom or a C₁-C₄-alkyl group,
• W is a hydrogen atom, a C₁-C₄-alkoxy group, a formyl group or a group 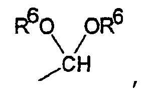
in which R⁶ is a C₁-C₄-alkyl group,
**B.** at least one 1-acylinodolin-3-one with the formula II or a derivative thereof or physiologically compatible salts thereof, 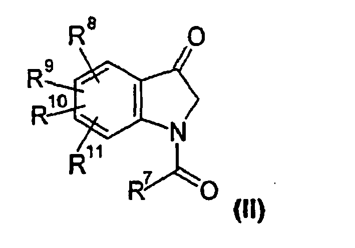 where
• R⁷ is a hydrogen atom, a C₁-C₄-alkyl group, an aryl group, an aryl-C₁-C₄-alkyl group or a heteroaryl group and
• R⁸, R⁹, R¹⁰ and R¹¹, in each case independently of one another, are a hydrogen atom, a halogen atom, a C₁-C₄-alkyl group, C₁-C₄-alkoxy group or C₁-C₄-hydroxyalkoxy group, a hydroxy group, nitro group, carboxy group, carboxylato group, C₁-C₄-alkoxycarbonyl group, an optionally substituted carbamoyl group, sulpho group, sulphato group, sulphamoyl group or amino group, which may be substituted by C₁-C₄-alkyl or C₁-C₄-hydroxyalkyl groups,
and customary cosmetic ingredients is applied to the fibres containing keratin, left on the fibres for a certain time, usually about 30 minutes, and then rinsed out again or washed out with a shampoo.

16. Method according to Claim 15, **characterized in that**, besides component A and component B, a further component
**C**. consisting of at least one compound with a primary or secondary amino group or hydroxy group chosen from primary or secondary aromatic amines, nitrogen-containing heterocyclic compounds and aromatic hydroxy compounds, amino acids, oligopeptides constructed from 2 to 9 amino acids and/or at least one CH-acidic compound or quaternary ammonium compound,
and customary cosmetic ingredients is applied to the fibres containing keratin, left on the fibres for a certain time, usually about 30 minutes, and then rinsed out again or washed out with a shampoo.

## Revendications

1. Agent pour teindre des fibres kératiniques, en particulier les cheveux humains, contenant
A. au moins un aldéhyde et/ou une cétone, choisis parmi les composés de formules la et lb, et/ou leurs dérivés carbonyles fonctionnels, et/ou les sels acceptables sur le plan physiologique de ceux-ci, 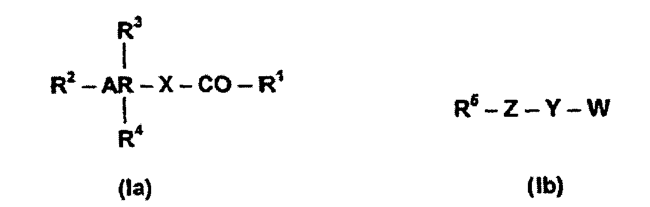 dans lesquelles
• AR représente un groupe benzène, naphtalène, pyridine, pyrimidine, pyrazine, pyrazidine, carbazole, pyrrole, pyrazole, furane, thiophène, 1,2,3-triazine, 1,3,5-triazine, quinoléine, isoquinoléine, indole, indoline, indolizine, indane, imidazole, 1,2,4-triazole, 1,2,3-triazole, tétrazole, benzimidazole, 1,3-thiazole, benzothiazole, indazole, benzoxazole, quinoxaline, quinazoline, quinolizine, cinnoline, acridine, julolidine, acénaphtène, fluorène, biphényle, diphénylméthane, benzophénone, éther diphénylique, azobenzène, chromone, coumarine, diphénylamine, stilbène, les substances N-hétéroaromatiques pouvant être également quaternisées,
• R¹ représente un atome d'hydrogène, un groupe alkyle en C₁ à C₄, acyle en C₁ à C₄, alcényle en C₂ à C₄, perfluoroalkyle en C₁ à C₄, un groupe aryle ou hétéroaryle éventuellement substitué,
• R², R³ et R⁴ représentent indépendamment l'un de l'autre, un atome d'hydrogène, un atome d'halogène, un groupe alkyle en C₁ à C₄, alcoxy en C₁ à C₄, aminoalkyle en C₁ à C₄, hydroxyalkyle en C₁ à C₄, un groupe acyle en C₁ à C₄, un groupe carboxy, carboxylato, sulfo, sulfato, alcényle en C₂ à C₆, aryle, aryl-(alkyle en C₁ à C₄), un groupe hydroxy, nitro, pyrrolidino, morpholino, pipéridino, amino, ou ammonio, ou 1-imidazol(in)io, les trois derniers groupes pouvant être substitués par des groupes alkyle en C₁ à C₄, carboxyalkyle en C₁ à C₄, hydroxyalkyle en C₁ à C₄, hydroxyalcoxy en C₁ à C₄, alcényle en C₂ à C₄, par des groupes benzyle éventuellement substitués, par des groupes sulfo-(alkyle en C₁ à C₄) ou hétérocycles-(alkyle en C₁ à C₄), où également deux des radicaux-X-CO-R¹, R², R³ et R⁴ peuvent former conjointement un cycle à 5, 6 ou 7 maillons condensés éventuellement substitué qui peut porter éventuellement un cycle aromatique condensé, le système AR pouvant porter d'autres substituants en fonction de la taille du cycle, lesquels peuvent représenter indépendamment l'un de l'autre les mêmes groupes que pour R², R³ et R⁴,
• X représente une liaison directe, un groupe carbonyle, méthylène, un groupe alcénylène en C₂ à C₆, alcadiénylène en C₄ à C₆, furylène, thiénylène, arylène, vinylène-arylène, vinylène-furylène, vinylène-thiénylène, éventuellement substitué, X pouvant également conjointement avec le groupe CO- R¹, former un cycle à 5, 6 ou 7 maillons, éventuellement substitué,
• R⁵ représente un atome d'hydrogène ou un groupe alkyle en C₁ à C₄,
• Z représente un groupe 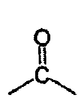
ou un groupe 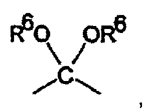 ou un groupe dans lequel R⁶ signifie un groupe alkyle en C₁ à C₄,
• Y représente une liaison directe, un groupe alkylène en C₁ à C₄, un groupe alcénylène en C₂ à C₄ ou alcadiénylène en C₄ à C₆ éventuellement substitué, ou un groupe CHOR^{6a}, dans lequel R^{6a} signifie un atome d'hydrogène ou un groupe alkyle en C₁ à C₄,
• W représente un atome d'hydrogène, un groupe alcoxy en C₁ à C₄, un groupe formyle, ou un groupe 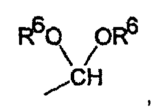
dans lequel R⁶ signifie un groupe alkyle en C₁ à C₄,
B. au moins une 1-acylindolin-3-one de formule II ou un dérivé de celle-ci ou ses sels acceptables sur le plan physiologique, 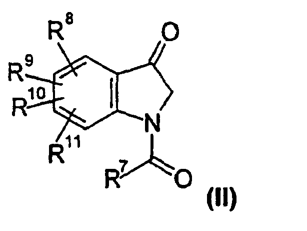 dans laquelle
• R⁷ représente un atome d'hydrogène, un groupe alkyle en C₁ à C₄, un groupe aryle, un groupe aryl-(alkyle en C₁ à C₄) ou un groupe hétéroaryle et
• R⁸, R⁹ et R¹⁰, et R¹¹ représentent chacun indépendamment l'un de l'autre un atome d'hydrogène, un atome d'halogène, un groupe alkyle en C₁ à C₄, alcoxy en C₁ à C₄, ou hydroxyalcoxy en C₁ à C₄, un groupe hydroxy, nitro, carboxy, carboxylato, (alcoxy en C₁ à C₄)carbonyle, un groupe carbamoyle, sulfo, sulfato, sulfamoyle, ou amino, éventuellement substitué, qui peut être substitué par des groupes alkyle en C₁ à C₄, ou hydroxyalkyle en C₁ à C₄.

2. Agent selon la revendication 1, **caractérisé en ce que** les composés de formule la sont choisis parmi les aldéhydes et/ou cétones, aromatiques et/ou hétéroaromatiques, en particulier parmi
- le 5-(4'-diméthylaminophényl)-penta-2,4-diénal, le 5-(4'-diéthylaminophényl)-penta-2,4-diénal, le 5-(4'-méthoxyphényl)-penta-2,4-diénal, le 5-(3',4'-diméthoxyphényl)-penta-2,4-diénal, le 5-(2',4'-diméthoxyphényl)-penta-2,4-diénal, le 5-(4'-pipéridinophényl)-penta-2,4-diénal, le 5-(4'-morpholinophényl)-penta-2,4-diénal, le 5-(4'-pyrrolidino-phényl)-penta-2,4-diénal, le 6-(4'-diméthylaminophényl)-hexa-2,4-dién-2-one, la 6-(4'-diéthylaminophényl)-hexa-2,4-dién-2-one, la 6-(4'méthoxyphényl)-hexa-2,4-dién-2-one, la 6-(3',4'-diméthoxyphényl)-hexa-2,4-dién-2-one, la 6-(2',4'-diméthoxyphényl)-hexa-2,4-dién-2-one, la 6-(4'-pipéridinophényl)-hexa-2,4-dién-2-one, la 6-(4'-morpholino-phényl)-hexa-2,4-dién-2-one, la 6-(4'-pyrrolidinophényl)-hexa-2,4-dién-2-one, le 5-(4'diméthylaminonapht-1-yl)-penta-2,4-diénal,
- le 2-nitropipéronal, le 5-nitropipéronal, le 6-nitropipéronal, le 5-hydroxy-2nitropipéronal, le 2-hydroxy-5-nitropipéronal, le 2-chloro-6-nitropipéronal, le 5-chloro-2-nitropipéronal, le 2,6-dinitropipéronal,
- le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 4-hydroxy-3-nitrobenzaldéhyde, le 5-hydroxy-2-nitrobenzaldéhyde, 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5-nitrobenzaldéhyde, le 4,5-diméthoxy-2-nitrobenzaldéhyde, la 5-nitrovanilline, le 3,5-dinitrosalicylaldéhyde, le 5-bromo-3-nitrosalicyl-aldéhyde, l'acide 3-nitro-4-formylbenzènesulfonique, le 4-nitro-1-naphtaldéhyde, le 2-nitrocinnamaldéhyde, le 3-nitrocinnamaldéhyde, le 4-nitrocinnamaldéhyde,
- les carbazole-aldéhydes ou les carbazole-cétones, en particulier le 9-méthyl-3carbazole-aldéhyde, le 9-éthyl-3-carbazolealdéhyde, le 3-acétylcarbazole, le 3,6-diacétyl-9-éthylcarbazole, le 3-acétyl-9-méthylcarbazole, le 1,4-diméthyl-3carbazole-aldéhyde, le 1,4,9-triméthyl-3-carbazole-aldéhyde,
- les sels de 4-triméthylammoniobenzaldéhyde, 4-benzyldiméthylammonio-benzaldéhyde, 4-triméthylammoniocinnamaldéhyde, 4-triméthylammonio-naphtaldéhyde, 2-méthoxy4-triméthylammoniobenzaldéhyde, N-(4'-acétylphényl)triméthylammonium, 4-(N,N-diéthyl)-N-méthylammonio)-benzaldéhyde, N-(4'-benzoylphényl)-triméthylammonium, N-(4'-benzoylphényl)-N,N-diéthylméthylammonium, N-(4'-formylphényl)-N-méthylpyrrolidinium, N-(4'-formylphényl)-N-méthylpipéridinium, N-(4'-formylphényl)-N-méthylmorpholinium, N-(4'-acétylphényl)-N-méthylmorpholinium, N-(4'-benzoylphényl)-N-méthylmorpholinium, 3-formyl-N-éthyl-N-méthylcarbazolium, 3-formyl-9,9-diméthylcarbazolium, 1-(4'-acétylphényl)-3-méthylimidazolium, 1-(4'-acétylphényl)-3-méthyl-2-imidazolinium, 1-(4'-benzoyl-phényl)-3-méthylimidazolium, 5-acétyl-1,3-diéthyl-2-méthylbenzimidazolium, 5-triméthylammonio-1-indanone, en particulier les benzènesulfonates, p-toluènesulfonates, méthanesulfonates, éthanesulfonates, propane-sulfonates, perchlorates, sulfates, chlorures, bromures, iodures, tétrachlorozincates, méthylsulfates, trifluorométhanesulfonates, hexafluorophosphates, tétrafluoroborates,
- les sels de formyl-1-méthylpyridinium, 2-formyl-1-méthylpyridinium, 4-formyl-1éthylpyridinium, 2-formyl-1-éthylpyridinium, 4-formyl-1-benzylpyridinium, 2-formyl-1-benzylpyridinium, 4-formyl-1,2-diméthyl-pyridinium, 4-formyl-1,3-diméthylpyridinium, 4-formyl-1-méthyl-quinoléinium, 2-formyl-1-méthylquinoléinium, 4-acétyl-1-méthyl-pyridinium, 2-acétyl-1-méthylpyridinium, 4-acétyl-1-méthylquinoléinium, 5-formyl-1-méthylquinoléinium, 6-formyl-1-méthylquinoléinium, 7-formyl-1-méthylquinoléinium, 8-formyl-1-méthylquinoléinium, 5-formyl-1-éthylquinoléinium, 6-formyl-1-éthylquinoléinium, 7-formyl-1-éthylquinoléinium, 8-formyl-1-éthylquinoléinium, 5-formyl-1-benzyl-quinoléinium, 6-formyl-1-benzyl-quinoléinium, 7-formyl-1-benzyl-quinoléinium, 8-formyl-1-benzylquinoléinium, 5-formyl-1-allyl-quinoléinium, 7-formyl-1-allylquinoléinium et 8-formyl-1-allylquinoléinium, de 5-acétyl-1-méthylquinoléinium, 6-acétyl-1-méthylquinoléinium, 7-acétyl-1-méthylquinoléinium, 8-acétyl-1-méthylquinoléinium, 5-acétyl-1-éthylquinoléinium, 6-acétyl-1-éthylquinoléinium, 7-acétyl-1-éthylquinoléinium, 8-acétyl-1éthylquinoléinium, 5-acétyl-1-benzylquinoléinium, 6-acétyl-1benzylquinoléinium, 7-acétyl-1-benzylquinoléinium, 8-acétyl-1-benzylquinoléinium, 5-acétyl-1-allylquinoléinium, 6-acétyl-1-allyl-quinoléinium, 7acétyl-1-allylquinoléinium et 8-acétyl-1-allyl-quinoléinium, de 9-formyl-10-méthyl-acridinium, 4-(2'-formylvinyl)-1-méthylpyridinium, 1,3-diméthyl-2-(4'-formylphényl)-benzimidazolinium, 1,3-diméthyl-2-(4'-formylphényl)-imidazolinium, 2-(4'-formylphényl)-3-méthylbenzothiazolium, 2-(4'-acétylphényl)-3-méthylbenzothiazolium, 2-(4'-formylphényl)-3-méthylbenzoxazolium, 2-(5-formyl-2-furyl)-3-méthylbenzothiazolium, 2-(5-formyl-2-thiényl)-3-méthylbenzothiazolium, 2-(3'-formylphényl)-3-méthylbenzothiazolium, 2-(4'-formylnapht-1-yl)-3-méthylbenzothiazolium, 5-chloro-2-(4'-formylphényl)-3-méthyl-benzothiazolium, 2-(4'-formylphényl)-3,5-diméthylbenzothiazolium, 1-méthyl-2-[2-(4'-formylphényl)-éthényl]-pyridinium, 1-méthyl-4-[2-(4'-acétylphényl)-éthényl]-pyridinium, 1-benzyl-4-[2-(4'-formylphényl)-éthényl]-pyridinium, 1-méthyl-4-[2-(4'-formylphényl)-éthényl]-pyridinium, 1-méthyl-2-[2-(4'-formylphényl)-éthényl]-pyridinium, 1-méthyl-4-[2-(4'-formylphényl)-éthényl]-quinoléinium, 1-méthyl-2-[2-(4'-formylphényl)-éthényl]-quinoléinium, 1-méthyl-2[2-(5-formyl-2-furyl)-éthényl]-quinoléinium, 1-méthyl-2-[2-(5-formyl-2-thiényl)-éthényl]-quinoléinium, 1-méthyl-2-[2-(4 - formylphényl)-éthényl]benzothiazolinium, 1,3-diméthyl-2-[2-(4'-formylphényl)-éthényl]-benzimidazolinium, 1,3-diméthyl-2-[2-(4'-formylphényl)-éthényl]-imidazolinium, 1-méthyl-5-oxo-indéno[1,2-b]-pyridinium(4-méthyl-4-azonio-9-fluorénone), 1-éthyl-5-oxo-indéno[1,2-b]-pyridinium(4-éthyl-4-azonio-9-fluorénone), 1-benzyl-5-oxo-indéno[1,2-b]-pyridinium(-4-benzyl-4-azonio-9-fluorénone), 2-méthyl-5-oxo-indéno[1,2-c]pyridinium, 2-méthyl-9-oxo-indéno[2,1-c]pyridinium, 1-méthyl-9-oxo-indéno[2,1-b]pyridinium, en particulier le benzènesulfonate, p-toluènesulfonate, méthanesulfonate, perchlorate, sulfate, chlorure, bromure, iodure, tétrachlorozincate, méthylsulfate, trifluorométhanesulfonate, tétrafluoroborate,
- le salicylaldéhyde, la vanilline, le 4-hydroxy-3-méthoxycinnamaldéhyde (coniférylaldéhyde), le 2,4-dihydroxybenzaldéhyde, le 4-diméthylamino-benzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, 4-pipéridinobenzaldéhyde, la 4-diméthylamino-acétophénone, le 4-hydroxynaphtaldéhyde, le 4-diméthylaminonaphtaldéhyde, la 4-diméthylaminobenzylidèneacétone, le 4-diméthylamino-cinnamaldéhyde, le 2-diméthylaminobenzaldéhyde, le 2-chloro-4-diméthylaminobenzaldéhyde, le 4-diméthylamino-2-méthylbenzaldéhyde, le trans-4-diéthylamino-cinnamaldéhyde, le 4-(dibutylamino)-benzaldéhyde, le 4-diphénylaminobenzaldéhyde, le 2,3,6,7-tétrahydro-1H,5H-benzo[ij]quinolizine-9-carboxaldéhyde, le 4-diméthylamino-2-méthoxybenzaldéhyde, le 2,3,6,7-tétrahydro-8-hydroxy-1H,5H-benzo[ij]quinolizine-9-carboxaldéhyde, le 4-(1-imidazolyl)-benzaldéhyde, le 2-morpholinobenzaldéhyde, l'indol-3-carboxaldéhyde, le 1-méthylindol-3-carboxaldéhyde, le N-éthylcarbazole-3carboxaldéhyde, la 2-formyl-méthylène-1,3,3-triméthylindoline (aldéhyde tribasique),
- le 1,3-diacetylbenzène, le 1,4-diacetylbenzène, le 1,3,5-triacétylbenzène, la 2-benzoyl-acétophénone, la 2-(4'-méthoxybenzoyl)-acétophénone, la 2-(2-furoyl)-acétophénone, la 2-(3-pyridoyl)-acétophénone, la 2-(2-pyridoyl)-acétophénone,
- la 1-phényl-1,2-propanedione, la 1-phényi-1,2-butanedione, la 1-(chloro-phényl)-1,2-propanedione, la 1-phényl-3,3-diméthyl-1,2-butanedione, le benzile, l'anisile, le salicile, le 5,5'-dibromosalicile, le 2,2'-furyle, le 2,2'-thiényle, le 2,2'-, 4,4'-pyridile, le 6,6'-diméthyl-4,4'-pyridile, le 4-hydroxy-, 4-méthoxy-, 4-chloro-, 4-méthyl-, 4-diméthylamino-, 4,4'-dihydroxy-, -diméthyl-, -dibromo-, -dichloro-, - bis-diméthylamino-, 2,4-dihydroxy-, 3,3'-diméthoxy, 2'-chloro-3,4-diméthoxy-, 3,4,5,3',4',5'-hexaméthoxy-benzile,
- les dérivés d'isatine, comme la 5-chloroisatine, la 5-méthoxyisatine, la 5-nitroisatine, la 6-nitroisatine, la 5-sulfoisatine, l'acide isatine-5-sulfonique, l'acide isatine-4-carboxylique et l'acide isatine-5-carboxylique,
- les dérivés d'isatine N-substitués, comme la N-méthylisatine, la N-(2'-hydroxyalkyl)isatine, la N-(2'-hydroxypropyl)-isatine, la N-(3'-hydroxy-propyl)-isatine, la N-(2',3'-dihydroxypropyl)-isatine, la N-(2'-sulfoéthyl)-isatine, la (3'-sulfopropyl)-isatine, la N-allylisatine, la N-vinylisatine, la N-benzylisatine, la N-(4'-méthoxybenzyl)-isatine, la N-(4'-carboxybenzyl)-isatine, la N-(4'-sulfobenzyl)-isatine, la diméthylaminoéthyl)-isatine, la N-(2'-pyrrolidinoéthyl)-isatine, la N-(2'-pipéridinoéthyl)-isatine, la (2'-morpholinoéthyl)-isatine, la N-(2'-furylméthyl)isatine, la N-(thién-2-ylméthyl)-isatine, la N-(pyrid-2-ylméthyl)-isatine, la N-(pyrid-3-ylméthyl)-isatine, la N-(pyrid-4-ylméthyl)-isatine, l'acide N-allylisatine-5-sulfonique, la 5-chloro-N-(2'-hydroxyéthyl)-isatine, la 5-méthyl-N-(2'-hydroxyéthyl)-isatine, la 5,7-dichloro-N-allylisatine, la 5-nitro-N-allylisatine, la N-hydroxyméthylisatine, la N-hydroxyméthyl-5-méthylisatine, la N-hydroxyméthyl-5-chloroisatine, la N-hydroxyméthyl-5-sulfoisatine, la N-hydroxyméthyl-5carboxyisatine, la N-hydroxyméthyl-5-nitroisatine, la N-hydroxyméthyl-5bromoisatine, la N-hydroxyméthyl-5-méthoxyisatine, la N-hydroxyméthyl-5,7-dichloroisatine, la N-diméthylaminométhylisatine, N-diéthylaminométhylisatine, la N-(bis-(2'-hydroxyéthyl)-aminométhyl)-isatine, la N-(2'-hydroxyéthylaminométhyl)-isatine, la N-(bis-(2'-hydroxypropyl)-aminométhyl)-isatine, la N-pyrrolidinométhylisatine, la N-pipéridinométhylisatine, la N-morpholinométhylisatine, la N-(1,2,4-triazolyl)-méthylisatine, la N-(1-imidazolyl)-méthylisatine, la N-carboxy-méthylaminométhylisatine, la N-(2-carboxyéthylaminométhyl)isatine, la N-(3'-carboxypropylaminométhyl)-isatine, la N-(bis-(2'-hydroxyéthyl)-aminométhyl)-5-méthylisatine, la N-pipéridinométhyl-5-chloroisatine, la N-(2'-sulfoéthylamino)-isatine, ainsi que les sels de métal alcalin et éventuellement d'ammonium des composés acides, la quinisatine et ses dérivés, comme la N-méthylquinisatine,
- l'acétophénone, la propiophénone, la 2-hydroxyacétophénone, la 3-hydroxyacétophénone, la 4-hydroxyacétophénone, la 2-hydroxypropiophénone, la 3-hydroxypropiophénone, la 4-hydroxypropiophénone, la 2-hydroxybutyrophénone, la 3-hydroxybutyrophénone, la 4-hydroxy-butyrophénone, la 2,4-dihydroxyacétophénone, la 2,5-dihydroxyacéto-phénone, la 2,6-dihydroxyacétophénone, la 2,3,4-trihydroxyacéto-phénone, la 3,4,5-trihydroxyacétophénone, la 2,4,6-trihydroxyacéto-phénone, la 2,4,6-triméthoxyacétophénone, la 3,4,5-triméthoxyacéto-phénone, le 3,4,5-triméthoxy-acétophénone-diéthylcétal, la 4-hydroxy-3-méthoxy-acétophénone, la 3,5-diméthoxy-4-hydroxy-acétophénone, la 4-amino-acétophénone, la 4-diméthylamino-acétophénone, la 4-morpholino-acétophénone, la 4-pipéridino-acétophénone, la 4-imidazolino-acétophénone, la 2-hydroxy-5-bromoacétophénone, la 4-hydroxy-3-nitroacétophénone, l'acide acétophénone-2-carboxylique, l'acide acétophénone-4-carboxylique, la benzophénone, la 4-hydroxybenzophénone, la 2-aminobenzophénone, la 4,4'-dihydroxy-benzo-phénone, la 2,4-dihydroxybenzophénone, la 2,4,4'-trihydroxybenzo-phénone, la 2,3,4-trihydroxybenzophénone, la 2-hydroxy-1-acéto-naphtone, la 1-hydroxy-2acétonaphtone, la chromone, l'acide chromone-2-carboxylique, la flavone, la 3-hydroxyflavone, la 3,5,7-trihydroxyflavone, !a 4',5,7-trihydroxyflavone, la 5,6,7-trihydroxyflavone, la quercétine, l'indanone, la 9-fluorénone, la 3-hydroxyfluorénone, l'anthrone, la 1,8-dihydroxyanthrone,
- les composés carbonyle hétérocycliques comme le 2-indolaldéhyde, le 3-indolaldéhyde, le 1-méthylindol-3-aldéhyde, le 2-méthylindol-3-aldéhyde, le 1-acétylindol-3-aldéhyde, le 3-acétylindole, le 1-méthyl-3-acétylindole, le 2-(1,3,3-triméthyl-2-indolinylidène)-acétaldéhyde, le 1-méthylpyrrol-2-aldéhyde, le 1-méthyl-2-acétylpyrrole, le 1-pyridin-aldéhyde, le 2-pyridinaldéhyde, le 3-pyridinaldéhyde, la 4-acétylpyridine, la 2-acétylpyridine,la 3-acétylpyridine, le pyridoxal, le quinoléine-3-aldéhyde, le quinoléine-4-aldéhyde, le antipyrine-4-aldéhyde, le furfural, le 5-nitrofurfural, la 2-thiénoyl-trifluoro-acétone, la chromone-3-aldéhyde, la 3-(5-nitro-2-furyl)-acroléine, la 3-(2-furyl)-acroléine, l'imidazol-2-aldéhyde,
- les dérivés d'indanone, comme par exemple, la 1,2-indanedione, la 2-oximo-1-indanone, l'indane-1,2,3-trione-2-oxime, le 5-méthoxyindane-1,2,3-trione-2-oxime, la 2-nitro-1,3-indanedione,
ainsi que des mélanges quelconques des composés précédents.

3. Agent selon la revendication 1, **caractérisé en ce que** les composés de formule Ib sont choisis parmi le dialdéhyde maléique, le dialdéhyde fumarique, l'ester triméthylique de l'acide orthoformique, l'ester triéthylique de l'acide orthoformique, le glyoxal, le dialdéhyde malonique, le malonyl-dialdéhyde-bis-diméthylacétal, le malonyl-dialdéhyde-bis-diéthylacétal, l'aldéhyde glutaconique ainsi que les sels métalliques et d'ammonium des dialdéhydes.

4. Agent selon l'une quelconque des revendications 1 à 3, **caractérisé en ce que** les composés de formule II sont choisis parmi la 1-acétylindolin-3-one, la 1-propionylindolin-3-one, la 1-acétyl-5-chloro-indolin-3-one, la 1-acétyl-5-bromo-indolin-3-one, la 1-acétyl-5-nitro-indolin-3-one, la 1-acétyl-5-carboxy-indolin-3-one, la 1-acétyl-5-éthoxycarbonyl-indolin-3-one, la 1-acétyl-5-méthoxycarbonyl-indolin-3-one, la 1-acétyl-5-méthyl-indolin-3-one, la 1-acétyl-5-méthoxy-indolin-3-one, le 1-acétyl-3-acétoxyindole, leurs produits d'addition d'acide compatibles avec la peau et des mélanges quelconques des composés précédents.

5. Agent selon l'une quelconque des revendications 1 à 4, **caractérisé en ce que** les aldéhydes et/ou les cétones de formules la et/ou lb et les 1-acylindolin-3-ones de formule II sont contenus à chaque fois en une quantité de 0,03 à 65 mmoles, en particulier de 1 à 40 mmoles, par rapport à 100 g de la teinture totale.

6. Agent selon l'une quelconque des revendications 1 à 5, **caractérisé en ce qu'**il contient en outre, au moins un composé C ayant un groupe amino primaire ou secondaire ou groupe hydroxy, choisi parmi les amines aromatiques primaires ou secondaires, les composés hétérocycliques azotés, et les composés hydroxyaromatiques, les acides aminés, les oligopeptides construits à partir de 2 à 9 acides aminés, et/ou un composé à groupe CH acide et/ou un composé ammonium quaternaire.

7. Agent selon la revendication 6 **caractérisé en ce que** le composé C est choisi parmi
les amines aromatiques primaires et secondaires comme la N,N-diméthyl-, N,N-diéthyl-, N-(2'-hydroxyéthyl)-N-éthyl-, N,N-Bis-(2'-hydroxyéthyl)-, N-(2'méthoxyéthyl-), 2,3-, 2,4-, 2,5-dichloro-p-phénylènediamine, la 2-chloro-p-phénylènediamine, le dibromhydrate de 2,5-dihydroxy-4-morpholinoaniline, le 2-, 3-, 4-aminophénol, 2-aminométhyl-4-aminophénol, 2-hydroxyméthyl-4-aminophénol, la o-, p-phénylènediamine, la o-toluylène-diamine, le 2,5-diaminotoluène, le 2,5-diamino-phénol, le 2,5-diamino-phénéthol, le 4-amino-3-méthylphénol, le 2-(2',5'-diaminophényl)-éthanol, le 2,4-diaminophénoxyéthanol, le 2-(2',5'-diaminophénoxy)-éthanol, la 4-méthyl-amino-, 3-amino-4-(2'-hydroxyéthyloxy)-, 3,4-méthylendiamino-, 3,4-méthylène-dioxyaniline, le 3-amino-2,4-dichloro-, 4-méthylamino-, 2-méthyl-5-amino-, 3-méthyl-4-amino-, 2-méthyl-5-(2'-hydroxyéthylamino)-, 6-méthyl-3-amino-2-chloro-, 2-méthyl-5-amino-4-chloro-, 3,4-méthylènedioxy-, 5-(2'-hydroxyéthylamino)-4-méthoxy-2-méthyl-, 4-amino-2-hydroxyméthyl-phénol, le 1,3-diamino-2,4-diméthoxybenzène, l'acide 2-, 3-, 4-aminobenzoïque, l'acide 2-, 3-, 4-amino-phénylacétique, l'acide 2,3-, 2,4-, 2,5-, 3,4-, 3,5-diaminobenzoïque, l'acide 4-, 5-aminosalicylique, l'acide 3-amino-4-hydroxy-, 4-amino-3-hydroxy-benzoïque, l'acide 2-, 3-, 4-aminobenzènesulfonique, l'acide-3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxy-naphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-, 1,2,4-triaminobenzène, le 1,2,4,5-tétraamino-benzène, le 2,4,5-triaminophénol, le pentaaminobenzène, l'hexaaminobenzène, le 2,4,6-triaminorésorcinol, le 4,5-diaminopyrocatéchol, le 4,6-diaminopyrogallol, le 3,5-diamino-4-hydroxypyrocatéchol, bis-(5'-amino-2'-hydroxyphényl)-méthane, les nitriles aromatiques, les composes amino contenant des groupes nitro, comme la 3-amino-6-méthylamino-2-nitro-pyridine, l'acide picramique, le chlorure de [8-[(4'-amino-2'-nitrophényl)azo]-7-hydroxynapht-2-yl]-triméthylammonium, le chlorure de [8-((4'-amino-3'-nitrophényl)azo)-7-hydroxy-napht-2-yl]-triméthyl-ammonium (Basic Brown 17), le 1-hydroxy-2-amino-4,6-dinitrobenzène, le 1-amino-2-nitro-4-bis-(2'-hydroxyéthyl)-aminobenzène, le 1-amino-2-(2'-hydroxy-éthyl)-amino-5-nitrophénol (HC Yellow N° 5), le 1-amino-2-nitro-4-(2'-hydroxy-éthyl)-aminobenzène (HC Red N° 7), la 2-chloro-5nitro-N-hydroxyéthyl-1,4-phénylènediamine, le 1-(2'-hydroxyéthyl)-amino-2-nitro4-amino-benzène (HC Red N° 3), le 4-amino-3-nitrophénol, le 4-amino-2-nitrophénol, la 6-nitro-o-toluidine, le 1-amino-3-méthyl-4-(2'-hydroxyéthyl)-amino-6-nitrobenzène (HC Violet N° 1), 1-amino-2-nitro-4-(2',3'-dihydroxypropyl)-amino-5-chlorobenzène (HC Red N° 10), l'acide 2-(4'-amino-2'-nitroanilino)-benzoïque, la 6-nitro-2,5-diaminopyridine, le 2-amino-6-chloro-4-nitrophénol, l'acide 1-amino-2-(3'nitrophénylazo)-7-phénylazo-8-naphtol-3,6-disulfonique, sel disodique (Acid blue N° 29), l'acide 1-amino-2-(2'-hydroxy-4'-nitrophénylazo)-8-naphtol-3,6-disulfonique, sel disodique (Palatinchrome green), l'acide 1-amino-2-(3'-chloro-2'-hydroxy-5'-nitrophénylazo)-8-naphtol-3,6-disulfonique, sel disodique (Gallion), l'acide 4-amino-4'-nitrostilbène-2,2'-disulfonique, sel disodique, le 2,4-diamino-3',5'-dinitro-2'-hydroxy-5-méthyl-azobenzène (Mordant brown 4), l'acide 4'-amino-4-nitrodiphénylamine-2-sulfonique, l'acide 4'-amino-3'-nitrobenzophénone-2-carboxylique, le 1-amino-4-nitro-2-(2-nitrobenzylidène-amino)-benzène, la 2-[2'-(diéthylamino)éthylamino]-5-nitroaniline, l'acide 3-amino-4-hydroxy-5-nitrobenzènesulfonique, le 3-amino-3'-nitrobiphényle, le 3-amino-4-nitroacenaphtène, le 2-amino-1-nitronaphtalène, le 5-amino-6-nitrobenzo-1,3-dioxol, les anilines, en particulier les anilines contentant des groupes nitro comme la 4-nitroaniline, la 2-nitroaniline, le 1,4-diamino-2-nitrobenzène, le 1,2-diamino-4-nitrobenzène, le 1-amino-2-méthyl-6-nitrobenzène, le 4-nitro-1,3-phénylènediamine, le 2-nitro-4-amino-1-(2'-hydroxyéthylamino)-benzène, le 2-nitro-1-amino-4-[bis-(2'-hydroxyéthyl)-amino]benzène, l'acide 4-amino-2-nitrodiphénylamine-2'-carboxylique, le 1-amino-5-chloro-4-(2'-hydroxyéthylamino)-2-nitrobenzène, les phénols ou anilines aromatiques avec un résidu aromatique supplémentaire, comme par exemple le 4,4'-diaminostilbène et son chlorhydrate, le sel monosodique ou disodique de l'acide 4,4'-diaminostilbène-2,2'-disulfonique, le 4-amino-4'-diméthylamino-stilbène et son chlorhydrate, le 4,4'-diaminodiphénylméthane, le sulfure, sulfoxyde de 4,4'-diaminodiphényle, la 4,4'-diaminodiphénylamine, l'acide 4,4'-diaminodiphénylamine-2-sulfonique, la 4,4'-diaminobenzophénone, le 4,4'-diaminodiphényléther, le tétrachlorhydrate de 3,3',4,4'-tétraaminodiphényle, la 3,3',4,4'-tétraaminobenzophénone, le 1,3-bis(2',4'-diaminophénoxy)-propane, le 1,8-bis-(2',5'-diaminophénoxy)-3,6-dioxaoctane, le 1,3-bis-(4'-aminophénylamino)propane, le 1,3-bis-(4'-aminophénylamino)-2-propanol, le 1,3-bis-[N-(4'aminophényl)-2-hydroxyéthylamino]-2-propanol, le trichlorhydrate de N,N-bis-[2-(4'-aminophénoxy)-éthyl]-méthylamine, la N-phényf-1,4-phénylènediamine, le 1,4-bis-(4'-aminophényl)-1,4-diazacycloheptane, les composés azotés hétérocycliques choisis dans le groupe formé par la 2-, 3-, 4-amino-, 2-amino-3-hydroxy-, 2,6-diamino-, 2,5diamino-, 2,3-diamino-, 2-diméthylamino-5-amino-, 2-méthylamino-3-amino-6-méthoxy-, 2,3-diamino-6-méthoxy-, 2,6-diméthoxy-3,5-diamino-, 2,4,5-triamino-, 2,6-dihydroxy-3,4-diméthylpyridine, la 2,4-dihydroxy-5,6-diamino-, 4,5,6-triamino-, 4-hydroxy-2,5,6-triamino-, 2-hydroxy-4,5,6-triamino-, 2,4,5,6-tétraamino-, 2-méthylamino-4,5,6-triamino-, 2,4-, 4,5-diamino-, 2-amino-4-méthoxy-6-méthyl-pyrimidine, le 3,5-diamino-pyrazole, le 3,5-diamino-1,2,4-triazole, le 3-amino-, 3-amino-5-hydroxypyrazole, le 1-phényl-4,5-diaminopyrazole, le 1-(2'-hydroxyéthyl)-4,5-diaminopyrazole, le 1-phényl-3-méthyl-4,5-diaminopyrazole, la 4-amino-2,3-diméthyl-1-phényl-3-pyrazolin-5-one (la 4-aminoantipyrine), la 2-, 3-, 8-aminoquinoléine, la 4-amino-quinaldine, l'acide 2-, 6-aminonicotinique, la 5-aminoisoquinoléine, le 5-, 6-aminoindazole, le 5-, 7-aminobenzimidazole, le 5-, 7-aminobenzothiazole, la 2,5-dihydroxy-4-morpholinoaniline, ainsi que les dérivés d'indole et d'indoline comme le 4-, 5-, 6-, 7-aminoindole, le 5,6-dihydroxyindole, la 5,6-dihydroxy-indoline et la 4-hydroxyindoline, les dérivés d'hydroxypyrimidine ainsi que les composés hydroxyaromatiques comme le 2-, 4-, 5-méthylrésorcinol, le 2,5-diméthylrésorcinol, le résorcinol, le 3-méthoxyphénol, la pyrocatechin, l'hydroquinone, le pyrogallol, la phloroglucine, l'hydroxyhydroquinone, le 2-, 3-, 4-méthoxy-, 3diméthylamino-, 2-(2'-hydroxyéthyl)-, 3,4-méthylendioxyphénol, l'acide 2,4-, 3,4-dihydroxybenzoïque, l'acide 2,4-, 3,4-dihydroxy-phénylacétique, l'acide gallique, l'acide 2,4,6-trihydroxybenzoïque, 2,4,6-trihydroxyacetophénone, le 2-, 4-chlororésorcinol, le 1-naphtol, le 1,5-, 2,3-, 2,7-dihydroxynaphtalène, l'acide 6-diméthylamino-4-hydroxy-2-naphtalènesulfonique, l'acide 3,6-dihydroxy-2,7-naphtalènesulfonique, les acides aminés choisis dans le groupe formé par l'arginine, l'histidine, la tyrosine, la phénylalanine, la DOPA (dihydroxyphénylalanine), l'ornithine, la proline, la lysine, le tryptophane, l'acide 6-aminocaproïque et la β-alanine,
les oligopeptides choisis parmi le glutathion ou les oligopeptides contenus dans les hydrolysats de collagène, de kératine, de caséine, d'élastine, de protéine de soja, de gluten du blé ou de protéine d'amande,
les composés à groupe CH acide choisis dans le groupe formé par l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylèneindoline (base de Fischer), l'iodure de 2,3-diméthyl-benzothiazolium, le p-toluènesulfonate de 2,3-diméthyl-benzothiazolium, le p-toluènesulfonate de 1,2-diméthylnaphto[1,2-d]thiazolium, le p-toluènesulfonate de 1-éthyl-2-méthylnaphto[1,2-d]thiazolium, la rhodanine, l'acide rhodanine-3-acétique, l'iodure de 1-méthyl-2-quinaldinium, l'iodure de 1-éthyl-2quinaldinium, l'iodure de 1,4-diméthylquinoléinium, l'acide barbiturique, l'acide thiobarbiturique, l'acide 1,3-diméthylthiobarbiturique, l'acide 1,3-diéthylthiobarbiturique, l'oxindole, l'acétate de 3-indoxyle, la 2-coumaranone, la 5-hydroxy-coumaranone, la 6-hydroxycoumaranone, la 1-méthyl-3-phényl-2-pyrazolinone, l'indane-1,3-dione, l'indane-1-one, le benzoylacétonitrile, le 1-cyano-méthylène-indane, la 1,3-diiminoisoindoline et le chlorhydrate de 2-amino4-imino-1,3-thiazoline,
les sels d'ammonium quaternaires choisis dans le groupe formé par le chlorure, bromure, iodure, hydrogénosulfate, (semi)sulfate, de tétraméthylammonium, de tétraéthylammonium, de tétrapropylammonium, de tétrabutylammonium, benzyltriméthylammonium, ainsi que le Polyquaternium 10.

8. Agent selon la revendication 7, **caractérisé en ce que** le composé supplémentaire est choisi dans le groupe formé par la N-(2'-hydroxyéthyl)-N-éthyl-, 2-chloro-p-phénylènediamine, la N,N-bis-(2'-hydroxyéthyl)-p-phénylène-diamine, le 4-aminophénol, le 2-amino-6-chloro-4-nitrophénol, la p-phénylène-diamine, le 2-(2',5'-diaminophényl)-éthanol, le 2,5-diaminotoluène, la 3,4-méthylènedioxyaniline, le 2-amino-4-(2'-hydroxyéthylamino)-anisole, le 2-(2',4'-diaminophénoxy)-éthanol, le 3-amino-2,4-dichloro-, 2-méthyl-5-amino-, 3-méthyl-4-amino-, 2-méthyl-5-(2'-hydroxyéthylamino)-, 2-méthyl-5-amino-4chloro-, 6-méthyl-3-amino-2-chloro-, 2-aminométhyl-4-aminophénol, le 2-diéthyl-aminométhyl-4-aminophénol, le 2-diméthylaminométhyl-4-aminophénol, le 2,6-dichloro-4-aminophénol, le 2-hydroxyméthyl-4-aminophénol, le 3,4-méthylènedioxyphénol, l'acide 3,4-diaminobenzoïque, la 2,5-diamino-, 2-diméthylamino-5-amino-, 3-amino-2-méthylamino-6-méthoxy-, 2,3-diamino-6-méthoxy-, 3,5-diamino-2,6-diméthoxy-, 2,6-dihydroxy-3,4-diméthylpyridine, 2-hydroxy-4,5,6-triamino-, 4-hydroxy-2,5,6-triamino-, 2,4,5,6-tétraamino-, 2-méthylamino-4,5,6-triamino-pyrimidine, le 3,5-diaminopyrazole, le 3-amino-5-hydroxypyrazole, le 4,5-diamino-1-(2'-hydroxyéthyl)-pyrazole, le 5,6-dihydroxyindole et la 5,6-dihydroxyindoline, le bromure de tétrabutylammonium, la β-alanine, la L-proline, la L-lysine, la DL-tyrosine ainsi qu'à chaque fois les sels acceptables sur le plan physiologique de ces composés, formés de préférence avec des acides inorganiques.

9. Agent selon l'une quelconque des revendications 1 à 8, **caractérisé en ce qu'**il contient des renforçateurs de couleur choisis dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxy-pyridine, la 4-hydroxy-pyridine, l'imidazole, le 1-méthyl-imidazole, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

10. Agent selon l'une quelconque des revendications 1 à 9, **caractérisé en ce qu'**il contient des colorants montant directement sur la fibre issus du groupe des nitrophénylène-diamines, des nitroamino-phénols, des anthraquinones, ou des indophénols, de préférence en une quantité de 0,01 à 20% en poids, par rapport à la teinture totale.

11. Agent selon l'une quelconque des revendications 1 à 10, **caractérisé en ce que**, des sels d'ammonium ou métalliques sont contenus, choisis dans le groupe formé par les formiates, les carbonates, les halogénures, les sulfates, les butyrates, les valériates, les caproates, les acétates, les lactates, les glycolates, les tartrates, les citrates, les gluconates, les propionates, les phosphates et les phosphonates de métaux alcalins comme le potassium, le sodium ou le lithium, de métaux alcalino-terreux comme le magnésium, le calcium, le strontium ou le baryum, ou d'aluminium, de manganèse, de fer, de cobalt, de cuivre ou de zinc.

12. Agent selon l'une quelconque des revendications 1 à 11, **caractérisé en ce qu'**il contient des agents d'oxydation, en particulier H₂O₂, en une quantité de 0,01 à 6% en poids, par rapport à la solution d'application.

13. Agent selon l'une quelconque des revendications 1 à 12, **caractérisé en ce qu'**il contient des tensio-actifs anioniques, zwitterioniques ou non ioniques.

14. Utilisation d'une combinaison constituée
A. d'au moins un aldéhyde et/ou d'une cétone, choisi parmi les composés de formules la et/ou Ib, et/ou leurs dérivés carbonyles fonctionnels, et/ou les sels acceptables sur le plan physiologique de ceux-ci, 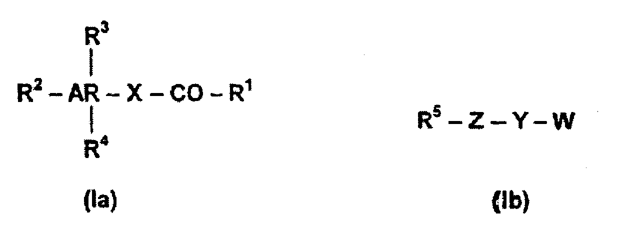 dans lesquelles
• AR représente un groupe benzène, napthalène, pyridine, pyrimidine, pyrazine, pyrazidine, carbazole, pyrrole, pyrazole, furane, thiophène, 1,2,3-triazine, 1,3,5-triazine, quinoléine, isoquinoléine, indole, indoline, indolizine, indane, imidazole, 1,2,4-triazole, 1,2,3-triazole, tétrazole, benzimidazole, 1,3-thiazole, benzothiazole, indazole, benzoxazole, quinoxaline, quinazoline, quinolizine, cinnoline, acridine, julolidine, acénaphtène, fluorène, biphényle, diméthylméthane, benzophénone, diphényléther, azobenzène, chromone, coumarine, diphénylamine, stilbène, les substances N-hétéroaromatiques, pouvant être également quaternisées,
• R¹ représente un atome d'hydrogène, un groupe alkyle en C₁ à C₄, acyle en C₁ à C₄, alcényle en C₂ à C₄, perfluoroalkyle en C₁ à C₄, un groupe aryle ou hétéroaryle éventuellement substitué,
• R², R³ et R⁴ représentent indépendamment l'un de l'autre un atome d'hydrogène, un atome d'halogène, un groupe alkyle en C₁ à C₄, alcoxy en C₁ à C₄, aminoalkyle en C₁ à C₄, hydroxyalkyle en C₁ à C₄, un groupe acyle en C₁ à C₄, un groupe carboxy, carboxylato, sulfo, sulfato, alcényle en C₂ à C₆, aryle, un groupe aryl-(alkyle en C₁ à C₄), un groupe hydroxy, nitro, pyrrolidino, morpholino, pipéridino, amino, ou ammonium, ou 1-imidazolinium, les trois derniers groupes pouvant être substitués par des groupes alkyle en C₁ à C₄, carboxyalkyle en C₁ à C₄, hydroxyalkyle en C₁ à C₄, hydroxyalcoxy en C₁ à C₄, alcényle en C₂ à C₄, par des groupes benzyle éventuellement substitués, par des groupes sulfo-(alkyle en C₁ à C₄) ou hétérocycle-(alkyle en C₁ à C₄), où également deux des radicaux -X-CO- R¹, R², R³ et R⁴ pouvent former conjointement un cycle condensé à 5, 6 ou 7 maillons, éventuellement substitué, qui peut porter éventuellement un cycle aromatique condensé, le système AR pouvant porter d'autres substituants en fonction de la taille du cycle, qui peuvent représenter indépendamment l'un de l'autre les mêmes groupes comme R², R³ et R⁴,
• X représente une liaison directe, un groupe carbonyle, méthylène, un groupe alcénylène en C₂ à C₆, alcadiénylène en C₄ à C₆, furylène, thiénylène, arylène, vinylènearylène, vinylènefurylène, vinylènethiénylène, éventuellement substitués, X pouvant former conjointement avec le groupe CO-R¹, également un cycle à 5, 6 ou 7 maillons éventuellement substitué,
• R⁵ représente un atome d'hydrogène ou un groupe alkyle en C₁ à C₄,
• Z représente un groupe 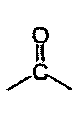
ou un groupe 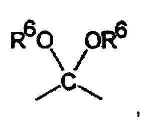 dans lequel R⁶ signifie un groupe alkyle en C₁ à C₄,
• Y représente une liaison directe, un groupe alkylène en C₁ à C₄, un groupe alcénylène en C₂ à C₄ ou alcadiénylène en C₄ à C₆ éventuellement substitué, ou un groupe CHOR^{6a}, dans lequel R^{6a} signifie un atome d'hydrogène ou un groupe alkyle en C₁ à C₄,
• W représente un atome d'hydrogène, un groupe alcoxy en C₁ à C₄, un groupe formyle, ou un groupe 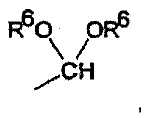
dans lequel R⁶ signifie un groupe alkyle en C₁ à C₄,
B. d'au moins une 1-acylindolin-3-one de formule Il ou d'un dérivé de celle-ci, ou de ses sels acceptables sur le plan physiologique, 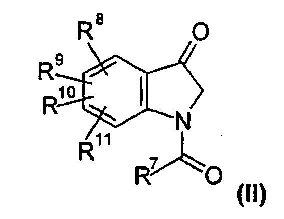 dans laquelle,
• R⁷ représente un atome d'hydrogène, un groupe alkyle en C₁ à C₄, un groupe aryle, un groupe aryl-(alkyle en C₁ à C₄), ou un groupe hétéroaryle, et
• R⁸, R⁹, R¹⁰ et R¹¹ représentent à chaque fois indépendamment l'un de l'autre, un atome d'hydrogène, un atome d'halogène, un groupe alkyle en C₁ à C₄, un groupe alcoxy en C₁ à C₄, ou hydroxyalcoxy en C₁ à C₄, un groupe hydroxy, nitro, carboxy, carboxylato, (alcoxy en C₁ à C₄)carbonyle, un groupe carbamoyle, sulfo, sulfato, sulfamoyle,
ou amino éventuellement substitué, qui peut être substitué par des groupes alkyle en C₁ à C₄, ou hydroxyalkyle en C₁ à C₄,
en tant que composant colorant dans des teintures capillaires par oxydation.

15. Procédé pour teindre les fibres kératiniques en particulier les cheveux humains, dans lequel une teinture, contenant
A. au moins une aldéhyde et/ou une cétone, choisi parmi les composés de formules la et lb, et/ou leurs dérivés carbonyles fonctionnels et/ou les sels acceptables sur le plan physiologique de ceux-ci, 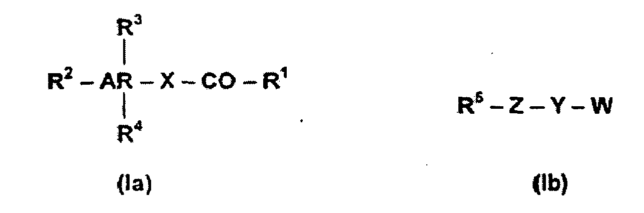 dans lesquelles
• AR représente un groupe benzène, napthalène, pyridine, pyrimidine, pyrazine, pyrazidine, carbazole, pyrrole, pyrazole, furane, thiophène, 1,2,3-triazine, 1,3,5-triazine, quinoléine, isoquinoléine, indole, indoline, indolizine, indane, imidazole, 1,2,4-triazole, 1,2,3-triazole, tétrazole, benzimidazole, 1,3-thiazole, benzothiazole, indazole, benzoxazole, quinoxaline, quinazoline, quinolizine, cinnoline, acridine, julolidine, acénaphtène, fluorène, biphényle, diméthylméthane, benzophénone, diphényléther, azobenzène, chromone, coumarine, diphénylamine, stilbène, les substances N-hétéroaromatiques, pouvant être également quaternisées,
• R¹ représente un atome d'hydrogène, un groupe alkyle en C₁ à C₄, acyle en C₁ à C₄, alcényle en C₂ à C₄, perfluoroalkyle en C₁ à C₄, un groupe aryle ou hétéroaryle éventuellement substitué,
• R², R³ et R⁴ représentent indépendamment l'un de l'autre un atome d'hydrogène, un atome d'halogène, un groupe alkyle en C₁ à C₄, alcoxy en C₁ à C₄, aminoalkyle en C₁ à C₄, hydroxyalkyle en C₁ à C₄, un groupe acyle en C₁ à C₄, un groupe carboxy, carboxylato, sulfo, sulfato, alcényle en C₂ à C₆, aryle, un groupe aryl-(alkyle en C₁ à C₄), un groupe hydroxy, nitro, pyrrolidino, morpholino, pipéridino, amino, ou ammonium, ou 1-imidazolinium, les trois derniers groupes pouvant être substitués par des groupes alkyle en C₁ à C₄, carboxyalkyle en C₁ à C₄, hydroxyalkyle en C₁ à C₄, hydroxyalcoxy en C₁ à C₄, alcényle en C₂ à C₄, par des groupes benzyle éventuellement substitués, par des groupes sulfo-(alkyle en C₁ à C₄) ou hétérocycle-(alkyle en C₁ à C₄), où également deux des radicaux -X-CO- R¹, R², R³ et R⁴ peuvent former conjointement un cycle condensé à 5, 6 ou 7 maillons, éventuellement substitué, qui peut porter également un noyau aromatique condensé, le système AR pouvant porter d'autres substituants en fonction de la taille du cycle, qui peuvent représenter indépendamment l'un de l'autre les mêmes groupes comme R², R³ et R⁴,
• X représente une liaison directe, un groupe carbonyle, méthylène, un groupe alcénylène en C₂ à C₆, alcadiénylène en C₄ à C₆, furylène, thiénylène, arylène, vinylènearylène, vinylènefurylène, vinylènethiénylène, éventuellement substitués, X pouvant former conjointement avec le groupe CO-R¹, également un cycle à 5, 6 ou 7 maillons éventuellement substitué,
• R⁵ représente un atome d'hydrogène ou un groupe alkyle en C₁ à C₄,
• Z représente un groupe 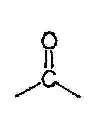
ou un groupe 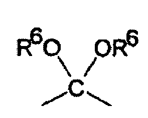 dans lequel R⁶ signifie un groupe alkyle en C₁ à C₄,
• Y représente une liaison directe, un groupe alkylène en C₁ à C₄, un groupe alcénylène en C₂ à C₄ ou alcadiénylène en C₄ à C₆ éventuellement substitué, ou un groupe CHOR^{6a}, dans lequel R^{6a} signifie un atome d'hydrogène ou un groupe alkyle en C₁ à C₄,
• W représente un atome d'hydrogène, un groupe alcoxy en C₁ à C₄, un groupe formyle, ou un groupe 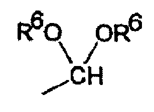
dans lequel R⁶ signifie un groupe alkyle en C₁ à C₄,
B. au moins une 1-acylindolin-3-one de formule Il ou un dérivé de celle-ci ou ses sels acceptables sur le plan physiologique, 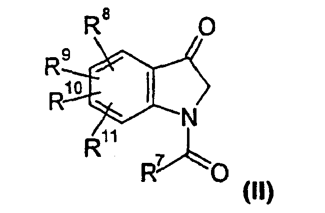 dans laquelle
• R⁷ représente un atome d'hydrogène, un groupe alkyle en C₁ à C₄, un groupe aryle, un groupe aryl-(alkyle en C₁ à C₄), ou un groupe hétéroaryle, et
• R⁸, R⁹ R¹⁰ et R¹¹ représentent à chaque fois indépendamment l'un de l'autre, un atome d'hydrogène, un atome d'halogène, un groupe alkyle en C₁ à C₄, un groupe alcoxy en C₁ à C₄, ou hydroxyalcoxy en C₁ à C₄, un groupe hydroxy, nitro, carboxy, carboxylato, (alcoxy en C₁ à C₄)carbonyle, un groupe carbamoyle, sulfo, sulfato, sulfamoyle,
ou amino éventuemment substitué, qui peut être substitué par des groupes alkyle en C₁ à C₄, ou hydroxyalkyle en C₁ à C₄,
ainsi que des ingrédients cosmétiques habituels, est appliquée sur les fibres kératiniques pendant quelques temps, habituellement pendant environ 30 minutes, est laissée sur la fibre, puis à nouveau rincée ou éliminée par lavage avec un shampoing.

16. Procédé selon la revendication 15, **caractérisé en ce que**, en plus du composant A et du composant B, un composant supplémentaire
C. constitué d'au moins un composé ayant un groupe amino primaire ou secondaire ou un groupe hydroxy, choisi parmi les amines aromatiques primaires ou secondaires, les composés hétérocycliques azotés, et les composés hydroxy aromatiques, les acides aminés, les oligopeptides construits à partir de 2 à 9 acides aminés, et/ou au moins un composé à groupe CH acide, ou un composé d'ammonium quaternaire,
ainsi que des ingrédients cosmétiques habituels, est appliqué sur les fibres kératiniques pendant quelques temps, habituellement pendant 30 minutes, est laissé sur la fibre, puis à nouveau rincé ou éliminé par lavage avec un shampoing.
